# EUROPEAN PATENT SPECIFICATION

(11) **EP 4 341 393 B1**
(45) Date of publication and mention of the grant of the patent: **05.08.2026**
(21) Application number: 22732994.3
(22) Date of filing: 07.06.2022
(51) Int. Cl.: C12N 9/68, A61K 38/48, A61K 38/00

(54) **PROCESS FOR ISOLATING PLASMINOGEN FROM A BLOOD PLASMA FRACTION**
VERFAHREN ZUR ISOLIERUNG VON PLASMINOGEN AUS EINER BLUTPLASMAFRAKTION
PROCÉDÉ POUR ISOLER LE PLASMINOGÈNE D'UNE FRACTION DE PLASMA SANGUIN

(30) Priority: 08.06.2021 EP 21178363
(43) Date of publication of application: 27.03.2024
(73) Proprietor: PreviPharma Consulting GmbH, 68167 Mannheim (DE); CSL Behring AG, 3014 Bern (CH)
(72) Inventor: MANDAGO, Maurice, 69250 Schönau (DE); WELZ, Ricarda, 69256 Mauer (DE); KIESSIG, Stephan T., 69168 Wiesloch (DE); GERBER, Simon, 3252 Worben (CH)
(74) Representative: Jacobi, Markus Alexander
(86) International application number: PCT/EP2022/065352
(87) International publication number: WO 2022/258590

(56) References cited:
- WO-A1-2018/162754
- US-A- 3 808 124
- US-A- 5 288 489
- US-B1- 6 207 066
- US-B2- 8 268 782

## Description

The present invention relates to a method for isolating plasminogen from a blood plasma fraction comprising dispersing a precipitate of a blood plasma fraction containing plasminogen in a basic aqueous buffer, separating solid parts thereof and removing at least parts of other proteins, and extracting the plasminogen with an acidic aqueous buffer.

Thrombotic events can cause serious health problems. For instance, coronary infarcts, stroke and pulmonary embolisms are some of the main causes of death in the developed world. There is a strong need to treat and prevent thrombotic events.

There are numerous cases of thrombotic events known. The formation of thrombotic events may, for example, occur in surgical interventions such as a vascular surgery (e.g., coronary bypass surgery), the presence of an endoprosthesis (i.e., insertion of foreign bodies/implants or transplants, e.g., blood vessel/vascular endoprosthesis), or a stenosis traumatic injury. A variety of further causes are known.

The treatment of thrombotic events is usually based on the administration of medicinal agents. In order to prevent the thrombus from enlarging, an anticoagulation is sought. Heparin preparations or inhibitors of Factor Xa are used. 4-Hydroxycoumarins such as phenprocoumone, warfarin or ethylbiscoumacetat are used for about three to six months. The use of cumarins typically requires regular blood tests and special attention, because the drugs prevent thrombosis, but also increase the propensity to bleed. When the growth of a thrombotic clot stops, the body can begin to clean up the damage. The body typically breaks down the clot and tries to get the veins free (re-vasculated) again. This typically takes several days, weeks or even months. In the breakdown of the thrombotic clot and the regeneration of the veins, substances may be released, which increase the coagulability of the blood. Most anticoagulatory drugs known in the art have severe drawbacks and often cause significant health risks. Problems regarding coagulation can be caused by, e.g., an unknown liver insufficiency or by an overdosing of the anticoagulatory drugs. The fibrinolytic site is currently not in focus. An observation of both sides can help to improve the results of the treatments. An improved treatment is desired.

As described in WO 2018/162754 and WO 2020/152322, it has been experimentally found that plasminogen, in particular Glu-plasminogen, can be effectively used for preventing or treating thrombotic events, in particular microthrombotic events, in a patient (WO 2018/162754). This can be of particular interest in patients suffering from inherent or acquired plasminogen deficiency. Glu-plasminogen is the native circulating precursor in the human plasma. Glu-plasminogen can be cleaved into Lys-plasminogen due to different isolation and purification processes. Treatment with Lys-plasminogen can result in undesired side effects such as bleeding. Glu-plasminogen has a significantly longer half-life and a well-predictable activity. Therefore, the purification of Glu-plasminogen is preferred.

It is of significant interest to obtain plasminogen, in particular Glu-plasminogen, in good yields and purity. Plasminogen is naturally occurring in blood. Blood and blood fractions also serve as significant sources for plasminogen and its subspecies. Accordingly, there is the need for methods for isolating plasminogen, in particular Glu-plasminogen, from blood fractions.

For decades, blood fractionation has been used for obtaining blood proteins such as albumin, immunoglobulin fractions, and hormones and other ingredients of blood such as platelets. Well-established blood fractionation processes are the Cohn process or Kistler-Nitschmann process. These processes, however, do typically not provide plasminogen, much less Glu-plasminogen, at high concentrations and yields. For technical and economic reasons, it may be beneficial to be able to obtain plasminogen, in particular Glu-plasminogen, from such established processes.

US 5,288,489 describes preparation of plasminogen from frozen Cohn fraction III or II/III paste, wherein the thawed material is suspended in phosphate buffered saline (PBS) containing 1 µM p-nitrophenyl-p-guanidinobenzoate, followed by centrifugation and filtration steps and subsequent chromatography via a lysineagarose material. Elution from the column is achieved by means of an increasing aminocaproic acid content in PBS. Alternatively, US 5,288,489 refers to a method wherein a similar process is conducted at pH 8.0. There does not seem to be a significant and purposeful alteration of pH in the methods of US 5,288,489.

US 3,808,124 discloses a process of purifying plasminogen from Cohn Fraction III. Plasminogen is adsorbed from Fraction III by affinity chromatography on lysine or arginine coupled agarose beads at cold room temperatures, e.g. 2°-7°C., using a soluble potassium phosphate buffer buffering the adsorption to a pH of approx. 7.4, an anti-clotting agent, such as citrates, and if desired a chelating agent, such as ethylene diamine tetra acetic acid (EDTA). The beads are washed and eluted with a phosphate buffer containing epsilon-amino caproic acid (EACA), also at approx. pH 7.4. Plasminogen is recovered, e. g. by precipitation with ammonium sulfate, and the beads regenerated with a concentrated urea solution.

WO 2002/095019 describes several stationary phases for chromatographic purification of plasmin and plasminogen from frozen cryoprecipitate of plasma which is contacted with a buffer of pH 7.0. Herein, it was found that tranexamic acid (TEA) immobilized on agarose is a usable column that also enables concomitant isolation of plasmin(ogen) and fibrinogen. The pH ranges as used are from neutral to basic. US 6,207,066 teaches the use of an aqueous basic buffer at pH 8 for removing blood plasma that does not contain plasminogen from a cartridge.

EP-B 1885485 teaches specific affinity chromatographic material and its use at pH 7.5 usable for purifying plasminogen from plasma. WO 2016/095013 teaches a pharmaceutic composition comprising plasminogen that has a pH of about 3 to about 10 and comprises a tonicity modifier and a stabilizing agent. The plasminogen is obtained from extracting plasminogen at a pH similar to the neutral pH.

US 8,268,782 describes mixing a caprylate precipitate obtained from the Cohn fractions II and III with a buffer of a pH of about 3.5 to about 10.5. In the more specific examples of the aforementioned patent, the caprylate precipitate has pH 5. Extraction is taught to be performed at a pH between neutral and pH 10.5, further adjusted to pH 7.5. It is shown that extraction at pH 3.5 is less efficient under the conditions used in US 8,268,782. Further, also the addition of lysine or epsilon amino caproic acid to such mixture and subsequent cation exchange chromatography is taught. The final plasminogen should be eluted and buffered at a pH of about 2.5 to about 4.

WO 2018/162754 describes a method for isolating Glu-plasminogen from plasma or a plasma fraction, comprising contacting the plasma or a plasma fraction with an anion exchanger and optionally adjusting the pH to a desired range.

The above methods enable at least partly isolation of plasminogen from plasma fractions, some even of plasminogen having a high Glu-plasminogen content. However, it is desired to improve the yields and purity further and to provide an efficient method that enables to obtain high quality plasminogen from a plasma fraction or even a side or waste fraction of a routine plasma fractionation process.

Surprisingly, it has been found that a method that comprises dispersing a precipitate of a blood plasma fraction comprising plasminogen in a basic aqueous buffer, separating solid parts thereof, and extracting the plasminogen with an acidic aqueous buffer, preferably comprising at least one compound of formula (I) or a salt or combination thereof, provides plasminogen.

Accordingly, disclosed is a method for isolating plasminogen from a blood plasma fraction, wherein said method comprises the following steps:
(i) dispersing a precipitate of blood plasma fraction comprising plasminogen in a basic aqueous buffer of pH 7 to 10;
(ii) incubating the dispersion obtained from step (i) to allow the dissolution of at least parts of the proteins and other impurities which are soluble in the basic aqueous buffer;
(iii) separating solid parts and liquid parts of the incubated dispersion of step (ii) from each other;
(iv) mixing the solid parts obtained from step (iii) with an acidic aqueous buffer of pH 2 to 6.6, wherein the acidic aqueous buffer further comprises dissolved lysine and/or at least one other compound of formula (I) or a salt thereof:

   (H₂N)ₙ-R-(A)ₘ (I),

   wherein:
   n is an integer of 1 or 2;
   m is an integer of 0, 1 or 2;
   A is at each occurrence independently from each other a carboxyl group, or an amino group;
   R is a linear or branched C₃-C₁₂-alkylene, a linear or branched C₃-C₁₂-heteroalkylene, a C₆-C₁₂-arylene optionally substituted by one or more halogens or one or more C₁-C₄-(hetero)alkyl residues, a C₃-C₁₂-heteroarylene optionally substituted by one or more halogens or one or more C₁-C₄-(hetero)alkyl residues, a C₃-C₁₂-alkylene-C₆-C₁₂-arylene optionally substituted by one or more halogens or one or more C₁-C₄-(hetero)alkyl residues, a C₃-C₁₂-alkylene-C₃-C₁₂-heteroarylene optionally substituted by one or more halogens or one or more C₁-C₄-(hetero)alkyl residues,
      and optionally incubating the mixture to allow the plasminogen to dissolve, and optionally removing solid parts; and
(v) obtaining a solution containing isolated plasminogen from step (iv).

A first aspect of the present invention relates to a method for isolating plasminogen from a blood plasma fraction, wherein said method comprises the following steps:
(i) dispersing a precipitate of blood plasma fraction comprising plasminogen in a basic aqueous buffer of pH 7 to 10;
(ii) incubating the dispersion obtained from step (i) to allow the dissolution of at least parts of the proteins and other impurities which are soluble in the basic aqueous buffer;
(iii) separating solid parts and liquid parts of the incubated dispersion of step (ii) from each other;
(iv) mixing the solid parts obtained from step (iii) with an acidic aqueous buffer of pH 2 to 6.6, wherein the acidic aqueous buffer further comprises dissolved lysine and/or at least one other compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, in particular 6-aminohexanoic acid,
   and optionally incubating the mixture to allow the plasminogen to dissolve, and optionally removing solid parts; and
(v) obtaining a solution containing isolated plasminogen from step (iv).

It was surprisingly found that the claimed method provides high yields of plasminogen, in particular Glu-plasminogen, of high purity. The method could be conducted with comparably low work efforts. Thus, the present invention is technically beneficial.

As used herein, a plasminogen may be any plasminogen or a derivative or combination of more types thereof (e.g., Glu-plasminogen, Lys-plasminogen and/or plasmin). The structure of such proteins and the function of the plasminogen/plasmin system are generally known well. Plasminogen in the sense of the present invention may be plasminogen of any species of interest. Preferably, plasminogen is of human or mammalian origin.

The terms "protein", "polypeptide" and "peptide" may be understood interchangeably throughout the invention in the broadest sense as any chemical entity mainly composed of amino acid residues and comprising at least twenty amino acid residues consecutively linked with another via amide bonds. It will be understood that a protein in the sense of the present invention may or may not be subjected to one or more posttranslational modification(s) and/or be conjugated with one or more non-amino acid moiety/moieties. The termini of the protein may, optionally, be capped by any means known in the art, such as, e.g., amidation, acetylation, methylation, acylation.

Posttranslational modifications are well-known in the art and may be but may not be limited to lipidation, phosphorylation, sulfatation, glycosylation, truncation, oxidation, reduction, decarboxylation, acetylation, amidation, deamidation, disulfide bond formation, amino acid addition, cofactor addition (e.g., biotinylation, heme addition, eicosanoid addition, steroid addition) and complexation of metal ions, non-metal ions, peptides or small molecules and addition of iron-sulphide clusters. Moreover, optionally, co-factors, in particular cyclic guanidinium monophosphate (cGMP), but optionally also such as, e.g., ATP, ADP, NAD⁺, NADH+H⁺, NADP⁺, NADPH+H⁺, metal ions, anions, lipids, etc. may be bound to the protein, irrespective on the biological influence of these co-factors. In the context of plasminogen, in particular, glycosylation and disulfide bonds may occur.

In a preferred embodiment, in the context of the present invention, plasminogen is Glu-plasminogen. Therefore, throughout the present invention, plasminogen may be specified as being Glu-plasminogen. An advantage of the method of the present invention is that two glycosylation patterns of Glu-plasminogen may be observable in the isolated product. This may reflect the natural Glu-plasminogen.

In an alternative embodiment, the plasminogen is Lys-plasminogen. In an alternative embodiment, the plasminogen is a combination of Glu-plasminogen and Lys-plasminogen. Herein, Lys-plasminogen and Glu-plasminogen may be combined in any variation (preferably with the majority being Glu-plasminogen). In an alternative preferred embodiment, the plasminogen is a combination of Glu-plasminogen and Lys-plasminogen and one or more other plasminogen derivatives. Herein, Lys-plasminogen and Glu-plasminogen and the one or more other plasminogen derivatives may be combined in any variation (preferably with the majority being Glu-plasminogen).

Glu-plasminogen is a plasma-derived proenzyme. It is known that Glu-plasminogen has (essentially) no proteolytic activity. Preferably, the plasminogen composition (preferably containing Glu-plasminogen) administered to the patient has (essentially) no proteolytic activity. Such (essential) absence in proteolytic activity may be understood in the broadest sense as generally understood by a person skilled in the art. Preferably, the enzymatic activity of Glu-plasminogen and/or the plasminogen composition (preferably containing Glu-plasminogen) administered to the patient is below 70 units (U, i.e., µmol substrate/min) per 1.0 g/L of total protein content, or below 50, below 25, below 10, below 9, below 8, below 7, below 6, below 5, below 2, below 1, below 0.5, below 0.1, or below 0.01 U per 1.0 g/L of total protein content. In this context, proteolytic activity may be determined by any means. For instance, it may be the activity determined by an S-2288 (Chromogenix) proteolytic activity assay. Alternatively, it may also be determined as the degradation of fibrin resulting in the generation of D-dimers. The specific enzyme activity of plasmin can be determined by measuring the generation of D-dimers from the degradation of fibrin. Preferably, proteolytic activity of Glu-plasminogen and/or the plasminogen composition (preferably containing Glu-plasminogen) administered to the patient is below the detection limit of the assay.

In a preferred embodiment, not being part of the claimed invention, the plasminogen composition administered to the patient contains plasminogen, in particular Glu-plasminogen, in a purity of at least 75% (w/w), at least 80% (w/w), at least 85% (w/w), at least 90% (w/w), at least 95% (w/w), at least 96% (w/w), at least 97% (w/w), at least 98% (w/w), or at least 99% (w/w), based on the total protein content.

In a preferred embodiment, the obtained plasminogen composition (preferably containing Glu-plasminogen composition) contains no or only a low endotoxin content of below 1 EU/mL, below 0.5 EU/mL, below 0.1 EU/mL, below 0.05 EU/mL, or below 0.01 EU/mL (as determined in a Limulus Amebocyte Lysate (LAL) endosafe endochrome assay according to European Pharmacopeia (version 5.0) chapter 2.6.14).

In a preferred embodiment, the plasminogen composition (preferably containing Glu-plasminogen) administered to the patient contains no or only a low immunoglobulin content of below 5 g/L, below 2 g/L, below 1 g/L, below 0.5 g/L, or below 0.1 g/L of immunoglobulins (determined in a nephelometric assay).

As used herein, other impurities, which are soluble in the basic aqueous buffer, may be any impurities such as one or more small molecular impurities such as, e.g., octanoic acid and/or one or more excipients, and/or may be one or more high-molecular weight impurities.

Blood plasma (also designated as "plasma", "plasm" or "blood plasm" etc.) may be obtained from any source. It may for instance be obtained from a blood preservation from which the cells have been removed. Blood plasma is also commercially available from various suppliers.

In the context of the present invention, the term "plasma fraction" may be understood in the broadest sense as any part separated from blood plasma that comprises Glu-plasminogen. The person skilled in the art knows several routes for preparing plasma fractions from blood plasma. One commonly known example is the Cohn process (also designated as Cohn method) based on freeze-thaw cycles and gradually increasing the concentration of ethanol in the solution.

A precipitate of a blood plasma fraction as used herein may be blood plasma or any fraction thereof, which comprises plasminogen. Preferably, the blood plasma fraction is obtained from a well-established blood fractionation processes such as, e.g., the Cohn or Kistler-Nitschmann process. In a preferred embodiment, the blood plasma fraction is selected from the group consisting of:
(a) cryo-poor plasma supernatant or cryo-poor plasma precipitate;
(b) a fraction of any of paste I, II or III of the Cohn process, or a combination of two or all thereof;
(c) a fraction of any of paste I, II or III of the Kistler-Nitschmann process, or a combination of two or all thereof; and
(d) a combination of two or all thereof.

In a preferred embodiment, the blood plasma fraction is selected from the group consisting of a fraction of paste I of the Cohn process, a fraction of paste II of the Cohn process, a fraction of paste III of the Cohn process, a fraction of pastes I and II of the Cohn process, a fraction of pastes I and III of the Cohn process, a fraction of pastes II and III of the Cohn process, a fraction of pastes I, II and III of the Cohn process. In a preferred embodiment, the blood plasma fraction is selected from the group consisting of a side or waste fraction of paste I of the Cohn process, a side or waste fraction of paste II of the Cohn process, a side or waste fraction of paste III of the Cohn process, a side or waste fraction of pastes I and II of the Cohn process, a side or waste fraction of pastes I and III of the Cohn process, a side or waste fraction of pastes II and III of the Cohn process, a side or waste fraction of pastes I, II and III of the Cohn process.

In a preferred embodiment, the blood plasma fraction is a fraction of pastes II and III of the Cohn process. In a preferred embodiment, the blood plasma fraction is a fraction of pastes I and II of the Cohn process. In a preferred embodiment, the blood plasma fraction is a side or waste fraction of pastes II and III of the Cohn process. In a preferred embodiment, the blood plasma fraction is a side or waste fraction of pastes I and II of the Cohn process.

In another preferred embodiment, the blood plasma fraction is selected from the group consisting of a fraction of paste I of the Kistler-Nitschmann process, a fraction of paste II of the Kistler-Nitschmann process, a fraction of paste III of the Kistler-Nitschmann process, a fraction of pastes I and II of the Kistler-Nitschmann process, a fraction of pastes I and III of the Kistler-Nitschmann process, a fraction of pastes II and III of the Kistler-Nitschmann process, a fraction of pastes I, II and III of the Kistler-Nitschmann process. In another preferred embodiment, the blood plasma fraction is selected from the group consisting of a side or waste fraction of paste I of the Kistler-Nitschmann process, a side or waste fraction of paste II of the Kistler-Nitschmann process, a side or waste fraction of paste III of the Kistler-Nitschmann process, a side or waste fraction of pastes I and II of the Kistler-Nitschmann process, a side or waste fraction of pastes I and III of the Kistler-Nitschmann process, a side or waste fraction of pastes II and III of the Kistler-Nitschmann process, a side or waste fraction of pastes I, II and III of the Kistler-Nitschmann process. In a preferred embodiment, the blood plasma fraction is Kistler-Nitschmann precipitate A (PPT-NA).

In a preferred embodiment, the blood plasma fraction is a fraction of pastes II and III of the Kistler-Nitschmann process. In a preferred embodiment, the blood plasma fraction is a fraction of pastes I and II of the Kistler-Nitschmann process. In a preferred embodiment, the blood plasma fraction is a side or waste fraction of pastes II and III of the Kistler-Nitschmann process. In a preferred embodiment, the blood plasma fraction is a side or waste fraction of pastes I and II of the Kistler-Nitschmann process.

In another preferred embodiment, the blood plasma fraction is selected from the group consisting of fraction of paste I of the Cohn process, fraction of paste II of the Cohn process, fraction of paste III of the Cohn process, fraction of pastes I and II of the Cohn process, fraction of pastes I and III of the Cohn process, fraction of pastes II and III of the Cohn process, fraction of pastes I, II and III of the Cohn process.

In a preferred embodiment, the blood plasma fraction is fraction of pastes II and III of the Cohn process. In a preferred embodiment, the blood plasma fraction is fraction of pastes I and II of the Cohn process.

In another preferred embodiment, the blood plasma fraction is selected from the group consisting of fraction of paste I of the Kistler-Nitschmann process, fraction of paste II of the Kistler-Nitschmann process, fraction of paste III of the Kistler-Nitschmann process, fraction of pastes I and II of the Kistler-Nitschmann process, fraction of pastes I and III of the Kistler-Nitschmann process, fraction of pastes II and III of the Kistler-Nitschmann process, fraction of pastes I, II and III of the Kistler-Nitschmann process.

In a preferred embodiment, the blood plasma fraction is fraction of pastes II and III of the Kistler-Nitschmann process. In a preferred embodiment, the blood plasma fraction is fraction of pastes I and II of the Kistler-Nitschmann process.

It will be understood that the precipitate of step (i) contains plasminogen, in particular Glu-plasminogen. Such precipitate may be obtainable (obtained) by any means. In a preferred embodiment, the precipitate is obtained from contacting the blood fraction comprising plasminogen with an organic solvent (e.g., octanoic acid, methanol, ethanol, pentanol, butanol, pentanol, hexanol, heptanol, octanol, phenol, acetone, etc.). In another preferred embodiment, the precipitate is obtained from contacting the blood fraction comprising plasminogen with a chaotropic compound (e.g., a chaotropic salt (e.g., a barium salt, a calcium salt, a magnesium salt, a chlorate), guanidinium hydrochloride, thiocyanate such as guanidinium thiocyanate, perchlorate, iodide, urea, thiourea, high concentrations of sodium chloride, etc.). Such organic solvent or chaotropic salt can also be designated as precipitating agent. In another preferred embodiment, the precipitate is obtained from cooling the blood fraction comprising plasminogen or subjecting the fraction to a freeze-thaw cycle.

A precipitate may be obtained by any means known in the art. In a preferred embodiment, the precipitate of blood plasma fraction comprising plasminogen is obtained from freezing and thawing the blood plasma or a fraction thereof (also: subjecting the blood plasma or a fraction thereof to a freeze-thaw cycle), change of temperature, change of pH, addition of at least one precipitating agent, or a combination of two or more thereof. In a preferred embodiment, the precipitating agent is selected from the group consisting of octanoic acid, ethanol, a salt, or polyethylene glycol, or a combination of two or more thereof to the blood plasma fraction.

In a preferred embodiment, the precipitate of blood plasma fraction comprising plasminogen is obtained from addition of octanoic acid to the blood plasma fraction. It will be understood that the term "octanoic acid" as used herein is understood in the broadest sense as any compound comprising an octanoate anion, i.e., including the free acid as well as a salt thereof.

In a preferred embodiment, the precipitate of blood plasma fraction comprising plasminogen is obtained from addition of octanoic acid to the blood plasma fraction, which is selected from the group consisting of:
(a) cryo-poor plasma supernatant or cryo-poor plasma precipitate;
(b) a side or waste fraction of any of paste I, II or III of the Cohn or Kistler-Nitschmann process, or a combination of two or all thereof;
(c) a fraction of any of paste I, II or III of the Cohn or Kistler-Nitschmann process, or a combination of two or all thereof; and
(d) a combination of two or all thereof.

The precipitate may be provided in any form. In a preferred embodiment, the precipitate of step (i) is obtained by filtration, preferably dead-end filtration. Then, it can be also designated as filter cake. In a preferred embodiment, the precipitate is an octanoic acid (OA) filter cake, i.e., a precipitate obtained by contacting a blood fraction comprising plasminogen with octanoic acid and subsequent filtration. Alternatively, the precipitate of step (i) is obtained by centrifugation.

The precipitate obtainable from a plasma fractionation process may be used directly when obtained from the plasma fractionation process or may be stored. Optionally, the method of the present invention may be coupled to a plasma fractionation process and conducted as part of the procedural flow (also designable as on-line). Alternatively, a stored precipitate may be used for the method of the present invention. Such stored precipitate may optionally be cooled or may be frozen and thawed when conducting the method of the present invention.

Dispersing may be carried out by any means. For instance, dispersing may be performed by adding the basic aqueous buffer and shaking, mixing (manually or via a stirrer, mixer or blender), or a combination thereof.

As used herein, the term "aqueous buffer" may be understood in the broadest sense as any buffer solution that mainly (i.e., of more than 50% by weight) comprises water.

In one alternative, the basic aqueous buffer solution does not comprise dissolved lysine, at least one compound of formula (I) or a salt or combination thereof. In a preferred embodiment, the basic aqueous buffer solution does not comprise dissolved lysine, and/or at least one other compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine.

The dispersion of step (i) may be obtained from any (precipitate) : (basic aqueous buffer) ratio. In a preferred embodiment, the (precipitate) : (basic aqueous buffer) weight ratio is in the range of from 20 : 1 to 1 : 20, of from 10 : 1 to 1 : 10, of from 2 : 1 to 1 : 20, of from 1 : 1 to 1 : 20, of from 1 : 2 to 1 : 10, of from 1 : 3 to 1 : 7, of from 1 : 4 to 1 : 6, or in the range of approximately 1 : 5.

Step (i) may be carried out at any conditions suitable for dispersing the precipitate in the basic aqueous buffer. Preferably, this step is carried out at a temperature in the range of between 0 °C and 30 °C, preferably in the range of between 4 °C and 25 °C, between 4 °C and 10 °C, or between 17 °C and 22 °C.

As noted above, the basic aqueous buffer of step (i) may have a pH in the range of pH 7 to 10. In a preferred embodiment, the basic aqueous buffer of step (i) is of pH 8 to 10, of pH 8.1 to 9.9, of pH 8.2 to 9.8, of pH 8.3 to 9.7, of pH 8.4 to 9.6, of pH 8.5 to 9.5, of pH 8.6 to 9.4, of pH 8.7 to 9.3, of pH 8.8 to 9.2, of pH 8.9 to 9.1, or of pH 9.

As noted above, the acidic aqueous buffer in step (iv) may have a pH in the range of pH 2 to 6.6. In a preferred embodiment, the acidic aqueous buffer in step (iv) is of pH 2 to 6, of pH 2.5 to 5.9, of pH 3 to 5.7, of pH 3.5 to 5.6, of pH 4 to 6, of pH 4.5 to 5.5, of pH 4.6 to 5.4, of pH 4.7 to 5.3, of pH 4.8 to 5.2, of pH 4.9 to 5.1 ,or of pH 5. Preferably, the pH of the acidic aqueous buffer in step (iv) is not above, more preferably below, pH 6.6, i.e. the isoelectric point of Glu-plasminogen.

In a preferred embodiment, the basic aqueous buffer of step (i) is of pH 8.1 to 9.9, of pH 8.2 to 9.8, of pH 8.3 to 9.7, of pH 8.4 to 9.6, of pH 8.5 to 9.5, of pH 8.6 to 9.4, of pH 8.7 to 9.3, of pH 8.8 to 9.2, of pH 8.9 to 9.1, or of pH 9; and
the acidic aqueous buffer in step (iv) is of pH 2.5 to 5.9, of pH 3 to 5.7, of pH 3.5 to 5.6, of pH 4 to 6, of pH 4.5 to 5.5, of pH 4.6 to 5.4, of pH 4.7 to 5.3, of pH 4.8 to 5.2, of pH 4.9 to 5.1,or of pH 5.

In a preferred embodiment, the pH-difference in between the basic aqueous buffer of step (i) and the aqueous buffer in step (iv) is at least 0.5, at least 1, at least 1.25, at least 1.5, at least 1.75, at least 2, at least 2.25, at least 2.5, at least 2.75, at least 3, at least 3.25, at least 3.5, at least 3.75, or at least 4. In a preferred embodiment, the pH-difference in between the basic aqueous buffer of step (i) and the aqueous buffer in step (iv) is between 0.5 and 10, between 1 and 9, between 2 and 8, between 2.5 and 7, between 3 and 6, between 3.5 and 5, or between 3.5 and 4.5.

It will be understood that the numerical values taught herein may be understood in the broadest sense as generally understood as rounded values that embrace the subsequent decimal number. For instance, pH 5 includes values from pH 4.5 to 5.4 as generally understood.

The step (ii) of incubating may be carried out at any conditions allowing the dissolution of at least parts of the proteins, which are soluble in the basic aqueous buffer. Preferably, this step is carried out at a temperature in the range of between 0 °C and 30 °C, preferably in the range of between 4 °C and 25 °C, between 4 °C and 10 °C, or between 17 °C and 22 °C. Temperature may optionally also alter over time.

The incubation time of step (ii) may be at any range allowing the dissolution of at least parts of the proteins, which are soluble in the basic aqueous buffer and essentially not destructing the plasminogen of interest. In a preferred embodiment, incubation of step (ii) is conducted for a time range of from 1 min to 48 hours, 5 min to 24 hours, 10 min to 12 hours, 15 min to 6 hours, 20 min to 2 hours, 25 min to 1 hour, 25 min to 45 min, or approximately 30 min.

When the incubation of step (ii) is completed, the solid parts and liquid parts of the incubated dispersion of step (ii) are separated from each other. This may be performed by any means. In a preferred embodiment, the step (iii) of separating solid parts and liquid parts from each other is obtained by means selected from the group consisting of filtration, including dead-end filtration, tangential flow filtration, or a combination thereof, dialysis, phase separation, preferably by sedimentation, and/or and centrifugation. In a preferred embodiment, the separation of step (iii) is achieved by filtration, preferably dead-end filtration. Then, the obtained separated solid parts can be also designated as filter cake.

In a preferred embodiment, the step (iii) of separating solid parts and liquid parts from each other is obtained by means of filtration, wherein before step (iii), at least one filtration aid is added to the dispersion. A filtration aid may be any filtration aid known in the art. In a preferred embodiment, the filtration aid is selected from the group consisting of polymers, preferably high-molecular weight polyethylene glycol (PEG), cellulose, or cellulose derivatives, diatomaceous earth, perlite, detergents (e.g., Tween-20), and combinations of two or more thereof. It will be understood that also a combination of two or more filtration aids may be used.

Step (iii) may be carried out at any conditions suitable for this purpose. Preferably, this step is carried out at a temperature in the range of between 0 °C and 30 °C, preferably in the range of between 4 °C and 25 °C, between 4 °C and 10 °C, or between 17 °C and 22 °C.

The steps (i) to (iii) may be conducted each once or may be repeated twice or more often.

Step (iv) of mixing the solid parts obtained from step (iii) with an acidic aqueous buffer may be conducted by any means. For instance, mixing may be performed by adding the acidic aqueous buffer and shaking, mixing (manually or via a stirrer, mixer or blender), or a combination thereof.

The one or more buffer agents used in any of the buffers used herein may be of any chemical nature. Preferably, the buffer agents are pharmaceutically acceptable buffer agents. In a preferred embodiment, the acidic aqueous buffer comprises formic acid, acetate, carbonate, hydrogen carbonate, and/or citrate, preferably sodium formate, sodium acetate or sodium citrate, in particular sodium acetate. In a preferred embodiment, the acidic aqueous buffer is an acetate buffer, in particular a sodium acetate buffer. For instance, acidic aqueous buffers as used herein may comprise a sodium acetate / glycine buffering system.

It will be understood that the acidic aqueous buffer may optionally also comprise one or more further ingredients. In a preferred embodiment, it comprises a watersoluble or emulsifiable polymer such as, e.g., polyethylene glycol (PEG), preferably having a mean molecular weight (determinable by size exclusion chromatography) of 200 to 35000 Da, 1000 to 100000 Da, 200 to 1000 Da, 500 to 5000 Da, 1000 to 10000 Da, 5000 to 50000 Da, or 10000 to 100000 Da.

The mixture of step (iv) may be obtained from any (solid part) : (acidic aqueous buffer) ratio. In a preferred embodiment, the (solid part) : (acidic aqueous buffer) weight ratio is in the range of from 20 : 1 to 1 : 20, of from 10 : 1 to 1 : 10, of from 2 : 1 to 1 : 20, of from 1 : 1 to 1 : 20, of from 1 : 1 to 1 : 10, of from 1 : 2 to 1 : 7, of from 1 : 2 to 1 : 5, or in the range of approximately 1 : 3.

Step (iv) may be carried out at any conditions suitable for this purpose. Preferably, this step is carried out at a temperature in the range of between 0 °C and 30 °C, preferably in the range of between 4 °C and 25 °C, between 4 °C and 10 °C, or between 17 °C and 22 °C.

In a preferred embodiment, the at least one compound selected from the group consisting of aminohexanoic acid, aminopentanoic acid, aminoheptanoic acid, aminooctanoic acid, aminononanoic acid, 6-diaminohexane, aminodecanoic acid, ornithine, aminomethyl benzoic acid, and oxalysine, in particular wherein the at least one compound of formula (I) is 6-aminohexanoic acid, also referred to as the dissolved other compound, is aminohexanoic acid.

In a preferred embodiment, the acidic aqueous buffer of step (iv) comprises lysine, in particular L-lysine.

In a preferred embodiment, the acidic aqueous buffer of step (iv) comprises 6-aminohexanoic acid.

Optionally, the mixture of step (iv) comprises one or more further ingredients. Optionally, one or more types of anion exchange resin may be added to the mixture of step (iv).

Optionally, step (iv) comprises incubating the mixture to allow the plasminogen to dissolve, and optionally removing solid parts.

In a preferred embodiment, the solid parts obtained in step (iv) are separated from the liquid fraction that mainly comprises the dissolved plasminogen. This may be performed by any means. In a preferred embodiment, the step (iv) comprises separating solid parts and liquid parts from each other by means selected from the group consisting of filtration, including dead-end filtration, tangential flow filtration, or a combination thereof, dialysis, phase separation, preferably by sedimentation, and/or and centrifugation. In a preferred embodiment, the optional separation of step (iv) is achieved by filtration, preferably dead-end filtration. Then, the obtained separated solid parts can be also designated as filter cake. Optionally, the washed solid parts are further washed with acidic aqueous buffer. For instance, half the volume used for mixing before may be used. This may be conducted once or more often. The obtained solutions may be united (also designable as pooling).

From the above procedural steps, a solution containing isolated plasminogen may be obtained. Such plasminogen has already particularly beneficial technical properties such as particularly high yield and purity. Nevertheless, the method can be even improved further by adding further steps.

In a preferred embodiment, step (v) further comprises one or more means which decrease the concentration of dissolved precipitating agent in the solution. This may be performed by any means, including multiphasic separation.

In a preferred embodiment, step (v) further comprises one or more means which decrease the concentration of dissolved precipitating agent in the solution, wherein the solution is contacted with at least one chromatography resin interacting with at least parts of the precipitating agent, in particular, octanoate anions, more preferably wherein said chromatography resin is an ion exchanger, in particular an anion exchanger. In a preferred embodiment, the dispersion from at least one anion exchanger is contacted, interacting with at least parts of the octanoate anions.

In a preferred embodiment, the solution containing isolated plasminogen obtained in step (v) is further subjected to at least one further step selected from the group consisting of:
contacting the solution with at least one ion exchanger (e.g., anion exchanger or cation exchanger) with or without size-excluding properties interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine or both;
contacting the solution with at least one size exclusion resin interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
contacting the solution with at least one hydrophobic or mixed-mode interaction chromatography resin interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
subjecting the solution to diafiltration removing the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
subjecting the solution to at least one precipitation-washing cycle;
chromatography based on a stationary phase comprising immobilized lysine and/or at least one immobilized compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
affinity chromatography selective for the plasminogen;
molecular size chromatography;
dialysis;
ultrafiltration, including dead-end ultrafiltration, tangential flow ultrafiltration, or a combination thereof; and
a combination of two or more thereof.

In a preferred embodiment, the solution containing isolated plasminogen obtained in step (v) is further subjected to at least the following further steps:
(vi) removing at least parts of the compound comprising one or more amino groups, in particular of the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, from the solution obtained in step (v), preferably by means of contacting the solution with at least one chromatography resin, interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, in particular wherein the chromatography resin is an ion exchanger (e.g., anion exchanger or cation exchanger, in particular anion exchanger); and
(vii) increasing the purity and/or the concentration of the plasminogen, preferably by means of performing chromatography based on a stationary phase comprising immobilized lysine and/or at least one immobilized compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine wherein preferably first a buffer allowing the interaction of the plasminogen with the stationary phase is used which is followed by eluting the plasminogen by means of a buffer that decreases the interaction of the plasminogen with the plasminogen; and
(viii) obtaining a solution containing isolated plasminogen from step (vii).

Steps (vi) and (vii) may be conducted in any buffer usable for this purpose and at any temperature.

Preferably, a buffer usable in step (vi) is an acidic aqueous buffer. For instance, such acidic aqueous buffer may be of pH 2.5 to 5.9, of pH 3 to 5.7, of pH 3.5 to 5.6, of pH 4 to 6, of pH 4.5 to 5.5, of pH 4.6 to 5.4, of pH 4.7 to 5.3, of pH 4.8 to 5.2, of pH 4.9 to 5.1, or of pH 5. Preferably, this step (vi) is carried out at a temperature in the range of between 0 °C and 30 °C, preferably in the range of between 4 °C and 25 °C, between 4 °C and 10 °C, or between 17 °C and 22 °C. Elution from a column may be performed by using a high salt buffer such as having a salt like sodium chloride at a molar concentration of between 0.5 and 2 M such as, e.g., approximately 1 M.

Preferably, a buffer usable in step (vii) is an acidic aqueous buffer. For instance, such acidic aqueous buffer may be of pH 2.5 to 5.9, of pH 3 to 5.7, of pH 3.5 to 5.6, of pH 4 to 6, of pH 4.5 to 5.5, of pH 4.6 to 5.4, of pH 4.7 to 5.3, of pH 4.8 to 5.2, of pH 4.9 to 5.1, of pH 3, or pH 4, or of pH 5. Preferably, this step (vii) is carried out at a temperature in the range of between 0 °C and 30 °C, preferably in the range of between 4 °C and 25 °C, between 4 °C and 10 °C, or between 17 °C and 22 °C. The stationary phase of step (vii) may, for instance, be lysine-conjugated Sepharose.

Optionally, the pH may be kept rather low upon elution such as in a pH range of between pH 2 and pH 5. This may stabilize the plasminogen, in particular Glu-plasminogen, from undesired degradation and may increase shelf life thereof.

In a preferred embodiment, the method of the present invention comprises the following steps:
(i) dispersing a precipitate of blood plasma fraction comprising plasminogen in a basic aqueous buffer of pH 7 to 10, preferably 8 to 10, more preferably pH 8.5 to 9.5;
(ii) incubating the dispersion obtained from step (i) to allow the dissolution of at least parts of the proteins which are soluble in the basic aqueous buffer;
(iii) separating solid parts and liquid parts of the incubated dispersion of step (ii) from each other by means of filtration, including dead-end filtration, tangential flow filtration, or a combination thereof, dialysis, phase separation, preferably by sedimentation, and/or centrifugation;
(iv) mixing the solid parts obtained from step (iii) with an acidic aqueous buffer of pH 2 to 6.6, preferably pH 4.5 to pH 5, wherein the acidic aqueous buffer further comprises dissolved lysine, at least one other compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, or a salt or combination thereof, and optionally incubating the mixture to allow the plasminogen to dissolve, and removing solid parts; and
(v) obtaining a solution containing isolated plasminogen from step (iv);
(vi) removing at least parts of the compound comprising one or more amino groups, in particular of the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine,, from the solution obtained in step (v), preferably by means of contacting the solution with at least one chromatography resin, interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine,, in particular wherein the chromatography resin is an ion exchanger; and
(vii) increasing the purity and/or the concentration of the plasminogen by means of performing chromatography based on a stationary phase comprising immobilized lysine and/or at least one immobilized compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, wherein first a buffer allowing the interaction of the plasminogen with the stationary phase is used which is followed by eluting the plasminogen by means of a buffer that decreases the interaction of the plasminogen with the plasminogen; and
(viii) obtaining a solution containing isolated plasminogen from step (vii).

The obtained plasminogen, in particular Glu-plasminogen, may optionally be formulated, stored or may be directly used for any purpose.

In a preferred embodiment, step (iv) and/or step (vi), as far as present, includes contacting the solution with at least one small pore anion exchange resin interacting with at least part of the precipitating agent, in particular octanoic acid.

In a preferred embodiment, such at least one small pore anion exchange resin interacting with at least part of the precipitating agent is a resin having a microporous structure such as, e.g. pores in the range of from 0.1 to 10 nm, from 1 to 100 nm, from 0.1 to 10 µm, or from 1 to 100 µm. In a preferred embodiment, such at least one small pore anion exchange resin interacting with at least part of the precipitating agent is a resin based on one or more styrene-based (co)polymers and may contain quaternary ammonium groups and/or sulfonate groups.

In a preferred embodiment, such at least one small pore anion exchange resin interacting with at least part of the precipitating agent is a Dowex resin, in particular Dowex 1x8 having 50 to 100 mesh (0.15 to 0.3 mm average particle size), 100 to 200 mesh (0.08 to 0.15 mm average particle size), or 200 to 400 mesh (0.04 to 0.08 mm average particle size).

For instance, it may be used for therapeutic uses. Then, the plasminogen, in particular Glu-plasminogen, may be administered to an individual for use in a method for treating or preventing one or more thrombotic events. Then, preferably, the pH or a solution is adapted to the desired range before administration. Optionally, one or more further pharmaceutically acceptable ingredients may be added. Pharmaceutical and medicinal uses of plasminogen, in particular Glu-plasminogen, are described in detail in WO 2018/162754 and WO 2020/152322.

When storage is desired, the obtained plasminogen, in particular Glu-plasminogen, may optionally be lyophilized (freeze-dried) or frozen.

It will be understood that the method of the present invention does not only allow the preparation of plasminogen, but also of one or more further components as desirable side products.

In a preferred embodiment, the method of the present invention is further characterized in that:
(a) the proteins which are at least partly dissolved in step (ii) are recovered, preferably wherein said proteins comprise one or more immunoglobulins, in particular one or more fractions of immunoglobulin G, which may be optionally isolated;
(b) the method further comprises a virus inactivation step of the fraction of interest;
(c) the pH of the solution containing isolated plasminogen is further adjusted to a desired range, optionally by exchanging the aqueous buffer;
(d) the method further comprises freeze drying or drying of the plasminogen; or
(e) a combination of two or more thereof applies.

As indicated above, the plasminogen, in particular Glu-plasminogen, obtainable (obtained) by the method of the present invention bears special structural characteristics as such, such as particularly high purity and optionally minor amounts of residuals of the agents used in the procedural steps.

Accordingly, further disclosed is a composition comprising a plasminogen obtainable from a method of the present invention wherein plasminogen, in particular Glu-plasminogen, makes up at least 70% (w/w) of the total protein content.

It will be understood that the embodiments and definitions as laid out in the context of the method above *mutatis mutandis* apply to the plasminogen obtainable therefrom.

Preferably, plasminogen, in particular Glu-plasminogen, makes up at least 75% (w/w), at least 80% (w/w), at least 85% (w/w), at least 90% (w/w), or at least 95% (w/w), of the total protein content.

The (Glu-)plasminogen obtainable (or obtained) from a method of the present invention may be provided in a pharmaceutical composition. Accordingly, it may be admixed with one or more pharmaceutically acceptable carriers. Accordingly, further disclosed is a pharmaceutical composition comprising (Glu-)plasminogen obtainable (or obtained) from a method of the present invention and one or more pharmaceutically acceptable carriers.

The terms "pharmaceutical composition" and "pharmaceutical formulation" may be understood interchangeably. As used herein, the terms "pharmaceutically acceptable carrier", "pharmaceutically acceptable excipient", "carrier" and "excipient" may be understood interchangeably in the broadest sense as any substance that may support the pharmacological acceptance of the plasminogen and the at least one plasminogen activator, respectively. Such pharmaceutical composition may be ready to use and may preferably be a liquid formulation, in particular an injection portion.

The storage form may also be liquid, but may also be a dried form (e.g. a powder such as a powder comprising dried or freeze-dried plasminogen and the at least one plasminogen activator, respectively) or may be a paste or syrup or the like. Optionally, a dried form, paste or syrup may be dissolved or emulsified prior to being administered to the patient.

A pharmaceutically acceptable carrier may exemplarily be selected from the list consisting of an aqueous buffer, saline, water, dimethyl sulfoxide (DMSO), ethanol, vegetable oil, paraffin oil or combinations of two or more thereof. Furthermore, the pharmaceutically acceptable carrier may optionally contain one or more detergent(s), one or more foaming agent(s) (e.g., sodium lauryl sulfate (SLS), sodium dodecyl sulfate (SDS)), one or more coloring agent(s) (e.g., food coloring), one or more vitamin(s), one or more salt(s) (e.g., sodium, potassium, calcium, zinc salts), one or more humectant(s) (e.g., sorbitol, glycerol, mannitol, propylene glycol, polydextrose), one or more enzyme(s), one or more preserving agent(s) (e.g., benzoic acid, methylparaben, one or more antioxidant(s), one or more herbal and plant extract(s), one or more stabilizing agent(s), one or more chelating agents (e.g., ethylenediaminetetraacetic acid (EDTA), and/or one or more uptake mediator(s) (e.g., polyethylene imine (PEI), a cell-penetrating peptide (CPP), a protein transduction domain (PTD), an antimicrobial peptide, etc.).

The present disclosure also relates to a dosage unit of the pharmaceutical composition usable in the context of the treatment or prevention of the present disclosure. Exemplarily, it may refer to a single dose container or to a multiple dosage form.

The present disclosure also relates to a plasminogen, in particular Glu-plasminogen, of the present invention or a pharmaceutical composition of the present invention for use as a medicament.

The present disclosure also relates to a plasminogen, in particular Glu-plasminogen, of the present invention or a pharmaceutical composition of the present invention for use in a method for treating or preventing a thrombotic event. Such treatment with plasminogen, in particular Glu-plasminogen, is described in detail in WO 2018/162754 and WO 2020/152322.

As indicated above, the use of a basic aqueous buffer before purifying plasminogen was found to be particularly beneficial for the purity of the obtainable plasminogen.

Accordingly, a further aspect of the present invention relates to the use of a basic aqueous buffer of pH 8 to 11 for removing proteins other than plasminogen from a precipitate of a blood plasma fraction comprising plasminogen, wherein the use comprises a method of the present invention.

It will be understood that the embodiments and definitions as laid out in the context of the method above *mutatis mutandis* apply to the use.

The following examples and figures are intended to provide illustrative embodiments of the present invention described and claimed herein. These examples are not intended to provide any limitation on the scope of the invented subject-matter. The following figures, examples and claims further illustrate the invention.

### Brief description of the Figures

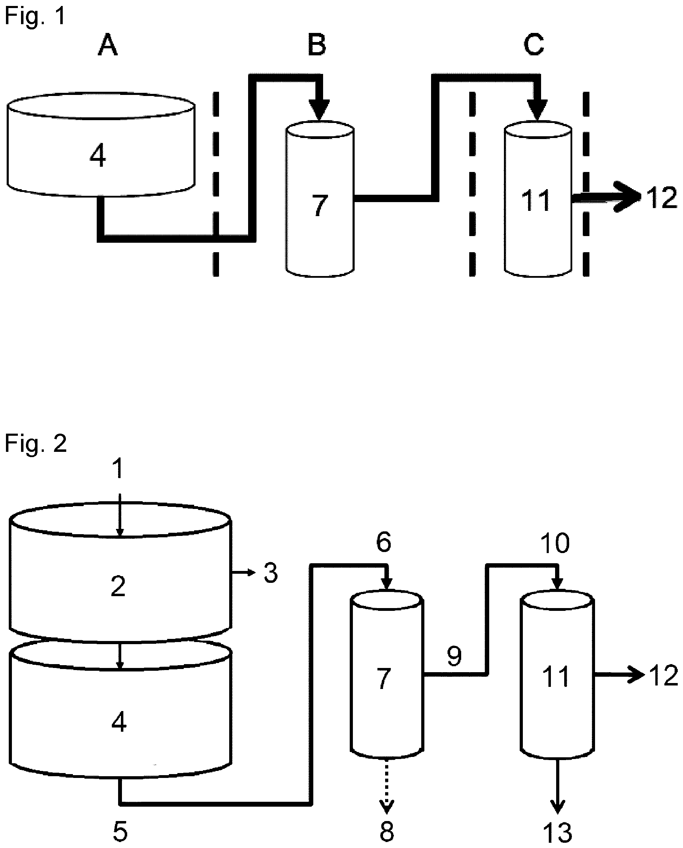

**Figure** 1 shows a flowchart of an example of the procedure of the present invention. A precipitate (e.g., an octanoic acid precipitate (OA-PPT)) of a blood plasma fraction comprising plasminogen may be provided as a filter cake and may be subjected to a resuspension step (A). This may provide a resuspension of a filter cake (4) in an acidic aqueous buffer. The obtained solution may be subjected to cation exchange chromatography (CEX) step (B) in a column (7) suitable for this purpose (e.g., Eshmuno CPX, Merck, Germany). Furthermore, an affinity chromatography step (C) is applied. For this purpose, a chromatography column (11) based on a stationary phase comprising immobilized lysine and/or at least one immobilized lysine analogue (e.g., based on lysine-conjugated Sepharose) may be used. The plasminogen product solution (12) may be obtained.
**Figure 2** shows an overview of another example of the procedure of the present invention. Herein, a precipitate of a blood plasma fraction (1) comprising plasminogen (e.g., an octanoic acid precipitate (OA-PPT) resuspension) may be dispersed in a basic aqueous buffer (2). For example, the weight ratio of (1) : (2) may be 1 : 5 and the pH may be set to pH 9 (e.g., pH 9.0). The dispersion may be incubated and filtered. This separates the filtrate comprising major impurities (total protein concentration may, for instance, be 3 to 6 g/L) from the precipitate. The remaining filter cake (4) may be dissolved in an acidic aqueous buffer comprising lysine and/or lysine analogue (e.g., 0.2 M sodium acetate containing 0.2 M 6-aminohexanoic acid (AHA) having pH 5 (e.g., pH 5.0) in a filter cake : buffer weight ratio of 1 : 3. The obtained solution (5) comprises solubilized plasminogen and may have a total protein content of <1 g/L. This solution (5) may be further diluted (e.g., in a ratio of 1 : 3 with an acidic buffer such as a buffer comprising 10 mM sodium acetate and 50 mM glycine at pH 5 (e.g., pH 5.0)). The obtained solution (6) may be subjected to a cation exchange chromatography (CEX) column (7) (e.g., Eshmuno CPX, Merck, Germany). Thereby, lysine and/or a lysine analogue (8) (e.g., 6-aminohexanoic acid) may be effectively removed from the solution. This may result in a solution (9) which comprises the plasminogen of interest. Herein, a high salt buffer may be used as elution buffer (e.g., comprising 50 mM sodium acetate, 50 mM glycine and 1 M sodium chloride, pH 5 (e.g., 5.0)) as used for elution. The solution (9) may be diluted in a further, preferably acidic, buffer (10) (e.g., comprising 50 mM sodium acetate, 50 mM glycine, pH 5 (e.g., 5.0)). The obtained solution may be subjected to a chromatography column (11) based on a stationary phase comprising immobilized lysine and/or at least one immobilized lysine analogue (e.g., based on lysine-conjugated Sepharose). Plasminogen of interest may be bound. The eluate (13) preferably does not contain detectable plasminogen contents. The plasminogen product solution (12) may be obtained, e.g., by means of an elution buffer comprising 50 mM citrate and 50 mM glycine, at pH 3 (e.g., pH 3.0).

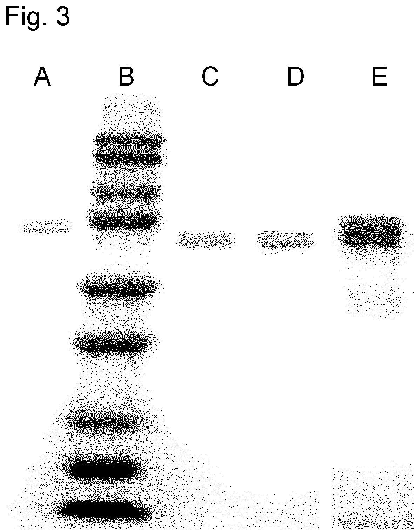

**Figure 3** shows an SDS-PAGE under non-reducing conditions with a total protein load of 2.5 µg/well. Lane A refers to a standard of Glu-plasminogen (0.6 µg/well). Lane B refers to an All Blue Precision Standard. Lane C refers to a standard of Lys-plasminogen (0.6 µg/well). Lane D refers to a standard of Lys-plasmin (0.6 µg/well). Lane E refers to an eluate from a lysine-conjugated Sepharose column.

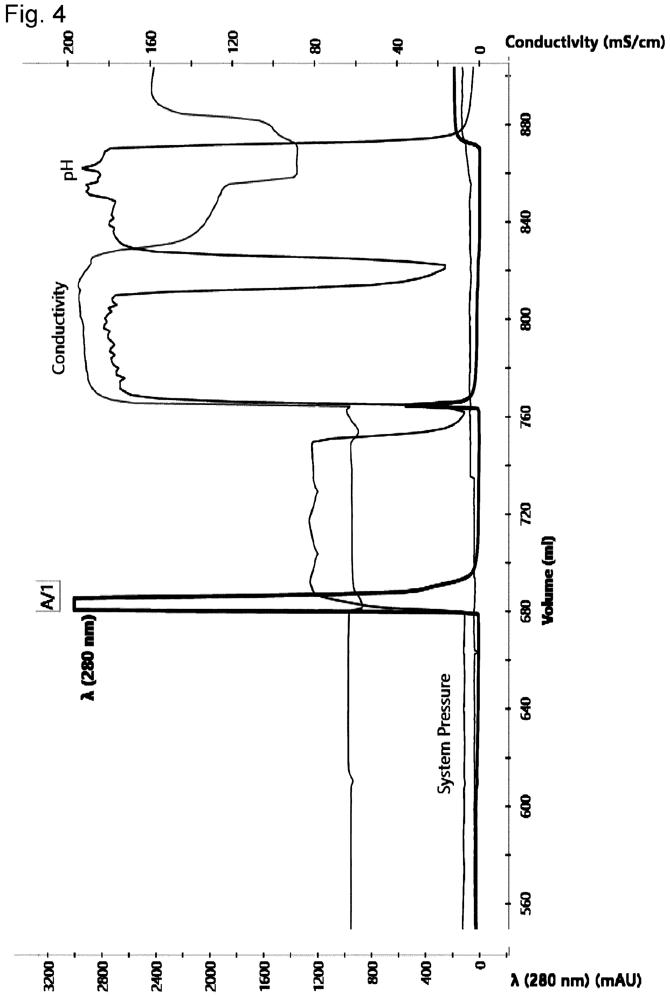

**Figure 4** shows a chromatogram of cation exchange (CEX) chromatography conducted with the resin Eshumuno CPX (Merck, Germany) as laid out in the experimental section below. There was no significant peak before peak A/1.

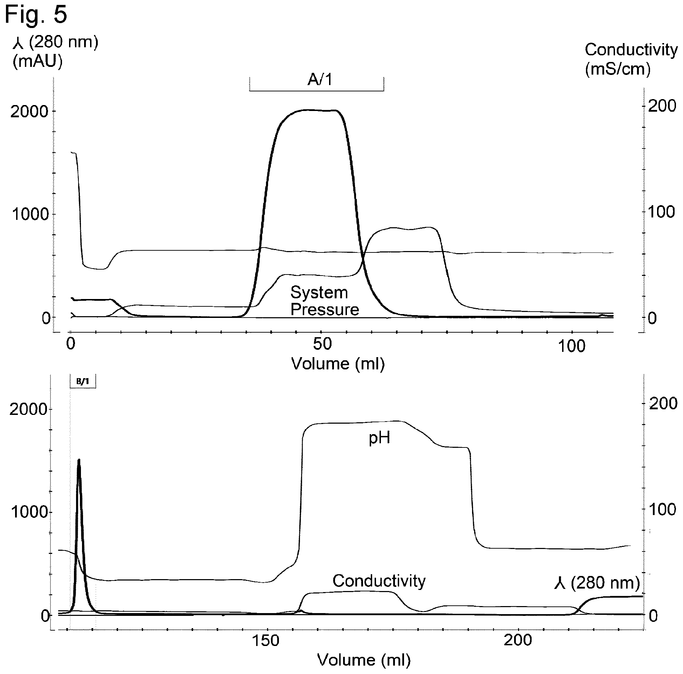

**Figure 5** shows a chromatogram of an affinity chromatography conducted with a column based on immobilized lysine as laid out in the experimental section below. There are two peaks A/1 and B/1.

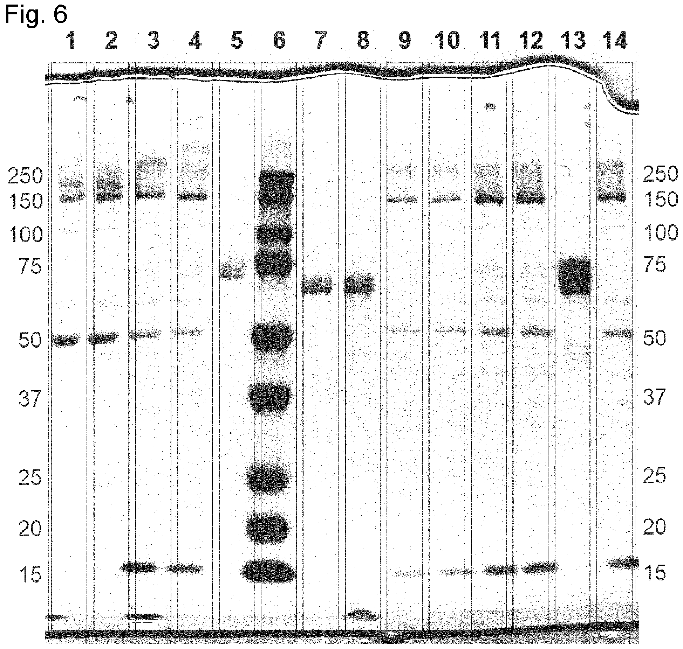

**Figure 6** shows an SDS-page gel provided as described in the experimental section below, wherein the lanes are the following:
   (1) first filtrate of the resuspension step, 2.5 µg/well;
   (2) filtrate from the first washing step of the resuspension step, 2.5 µg/well;
   (3) filtrate from second washing step of the resuspension step, 2.5 µg/well;
   (4) filtrate from second washing step of the resuspension, original concentration;
   (5) Glu-plasminogen standard, 6 µg/well;
   (6) All Blue Precision Protein Standard;
   (7) Lys-plasminogen standard, 6 µg/well;
   (8) Lys-plasmin standard, 0.6 µg/well;
   (9) feed of the CEX chromatography before loading of the column, 1:3 diluted second resuspension filtrate;
   (10) feed of the CEX chromatography after loading of the column, 1:3 diluted second resuspension filtrate;
   (11) feed of affinity chromatography before loading of the column, 1:2 diluted CEX eluate, 2.5 µg/well;
   (12) feed of affinity chromatography after loading of the column, 1:2 diluted CEX eluate, 2.5 µg/well;
   (13) eluate of the affinity chromatography, 2.5 µg/well; and
   (14) flow-through of the affinity chromatography, 2.5 µg/well.

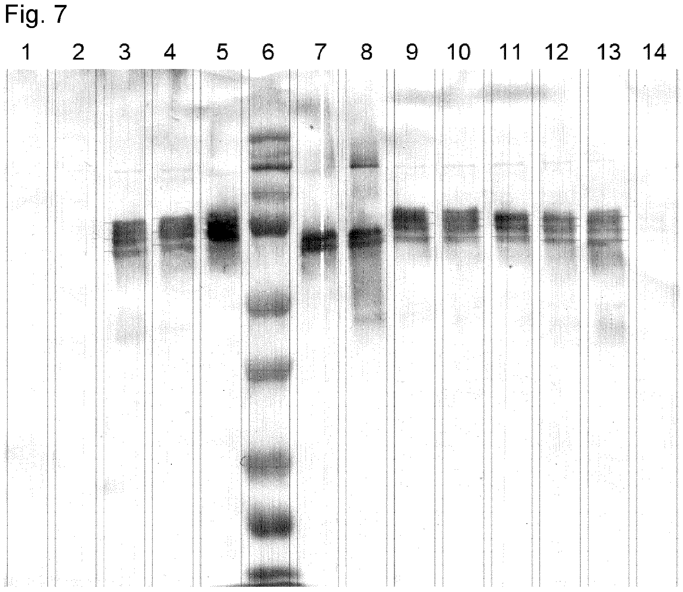

**Figure 7** shows a Western blot gel provided as described in the experimental section below, wherein the lanes are the following:
   (1) first filtrate of the resuspension step, 1 µg/well;
   (2) filtrate from the first washing step of the resuspension step, 1 µg/well;
   (3) filtrate from second washing step of the resuspension step, 1 µg/well;
   (4) filtrate from second washing step of the resuspension, 1 µg/well;
   (5) Glu-plasminogen standard, 0.09 µg/well;
   (6) All Blue Precision Protein Standard;
   (7) Lys-plasminogen standard, 6 µg/well;
   (8) Lys-plasmin standard, 0.9 µg/well;
   (9) feed of the CEX chromatography before loading of the column, 1:3 diluted second resuspension filtrate, 1 µg/well;
   (10) feed of the CEX chromatography after loading of the column, 1:3 diluted second resuspension filtrate, 1 µg/well;
   (11) feed of affinity chromatography before loading of the column, 1:2 diluted CEX eluate, 1 µg/well;
   (12) feed of affinity chromatography after loading of the column, 1:2 diluted CEX eluate, 1 µg/well;
   (13) eluate of the affinity chromatography, 0.15 µg/well; and
   (14) flow-through of the affinity chromatography, 1 µg/well.

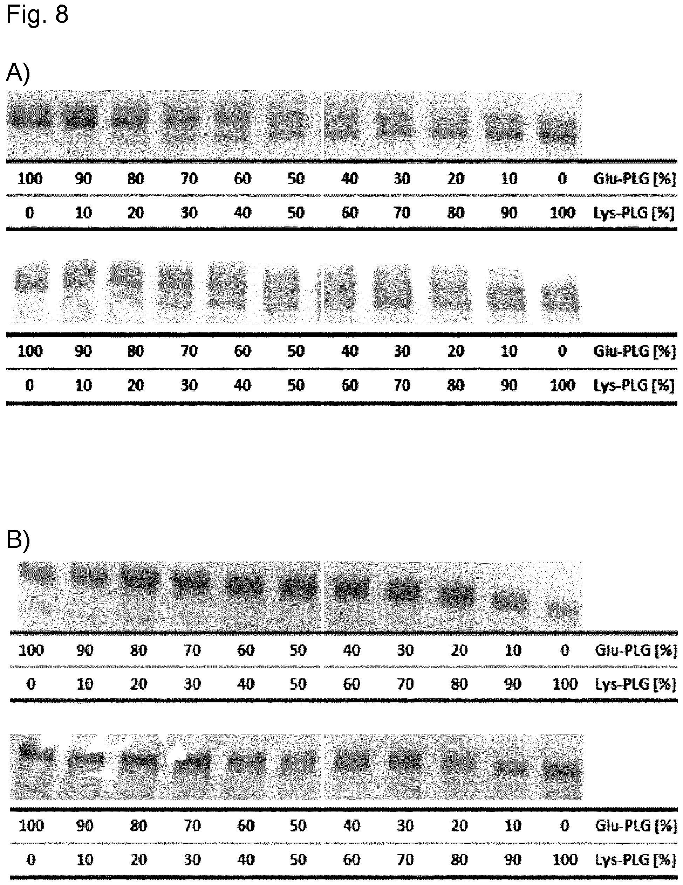

**Figure 8** shows bands in an SDS-page gel showing the Glu-plasminogen in comparison to Lys-plasminogen under non-reductive conditions (A) and under reductive conditions (B). It is visible that Lys-plasminogen is stronger visible.

### Examples

### Example of a method for isolating (Glu-)plasminogen from a blood plasma fraction

### I) Materials and methods

Precipitates obtained from the precipitation of a blood plasma fraction containing Glu-plasminogen with octanoic acid (OA) (OA-precipitates, OA-PPT) were obtained from a standard blood fractionating process. For this purpose, OA-PPTs from a Kistler-Nitschmann process (Kistler-Nitschmann precipitate A, PPT-NA) were used.

In further comparative examples, OA-precipitates from a Kistler-Cohn process (Kistler-Cohn fractions I+II+III) were used, which resulted in comparable results. Filter cakes of the OA-precipitates of 180 g were used. In further comparative examples, OA-precipitates of 360 g were used, which resulted in comparable results.

### a) Dispersing the precipitate in a basic aqueous buffer

A filter cake of an OA-precipitate was dispersed in a basic aqueous buffer (containing 0.2 M sodium acetate and 3.5% (w/v) of PEG 2000, the generated suspension was adjusted to a pH of 9.0) at a mass ratio of filter cake : buffer in the preferable range of 1 : 5 (also range of 1:1 up to at least 1:10 were exemplarily used in alternative examples).

### b) Incubation of the dispersion

The dispersion was incubated and stirred for 1 hour under moderate cooled conditions (tempering unit, Lauda, Germany). A filter aid (5 g/kg Harborlite 900, Imerys Filtration France S.A.S.) was added and further incubated and stirred for 30 min at room temperature.

### c) Separation of solid parts from liquid parts of the incubated dispersion

The dispersion of containing the filter aid was filtered by means of a standard filter (PuraFix CH 9 P, Filtrox AG, Switzerland). The filter cake was washed with up to half of the buffer volume used for dispersing the precipitate. Filtration time was adjusted to the individual experiment. It was pursued until filtration was completed. Typically, it was in the range of approximately 22 min at 73 kg/m³.

It was surprisingly found that the use of a basic aqueous buffer for dispersing the precipitate followed by filtration enabled efficient essential removal of impurities such as, e.g., numerous proteins other than (Glu-)plasminogen, fatty acids, PEG 2000, and residuals of octanoic acid. Impurities could be removed in the filtrate. The remaining filter cake contained (Glu-)plasminogen.

### d) Solubilization of (Glu-)plasminogen

The filter cake containing (Glu-)plasminogen was mixed with an acidic aqueous buffer (containing 0.2 M sodium acetate and 0.2 M lysine analogue 6-aminohexanoic acid, adjusted to pH 5.0) at a mass ratio of filter cake : buffer in the preferable range of 1 : 3 (also range of 1:1 up to at least 1:10 were exemplarily used in alternative examples). 30 g Dowex 1x8 (DuPont Dow Chemicals, USA) per 180 g OA-precipitate were added.

The mixture was incubated for 1.5 hours under moderate cooled conditions (tempering unit, Lauda, Germany). Then, residuals were removed by filtration by means of a standard filter (PuraFix CH 9 P, Filtrox AG, Switzerland). Filtration time was adjusted to the individual experiment. It was pursued until filtration was completed. Typically, it was in the range of approximately 6 min, but was adapted to the filter load. The filter cake was washed in order to remove residual dissolved fractions. The (Glu-)plasminogen essentially remained in the solution. In order to improve yields, the filter residuals were washed with up to half the volume of the acidic aqueous buffer used for solubilization. The two solutions were subsequently combined prior to further processing.

### e) Cation exchange (CEX) chromatography

The solutions containing (Glu-)plasminogen obtained from the above were diluted in an acidic aqueous buffer (10 mM sodium acetate, 50 mM glycine, adjusted to pH 5.0) in a volume ratio 1 : 3 (in further comparative experiments, broader ranges were used).

The strong cation exchange (CEX) chromatography resin Eshumuno CPX (Merck, Germany) was used as stationary phase. This was used on an NGC Chromatography System (BioRad, USA) equipped with a 12 mL chromatography column (126 x 11 mm) using a flow-through of 2.4 mL/min (5 min contact time). This column was equilibrated by an equilibrating buffer (200 mM sodium acetate, adjusted to pH 5.0). The diluted solutions containing (Glu-)plasminogen were loaded. Then, the column was washed with a wash buffer (50 mM sodium acetate, 50 mM glycine, adjusted to pH 5.0). Finally, the fraction containing (Glu-)plasminogen was eluted by an elution high salt buffer (50 mM sodium acetate, 50 mM glycine, 1 M NaCl, adjusted to pH 5.0).

It was found that the lysine analogue 6-aminohexanoic acid could effectively be removed from the solution.

### f) Chromatography with lysine-conjugated sepharose

The solutions containing (Glu-)plasminogen obtained from the above were diluted in an acidic aqueous buffer (50 mM sodium acetate, 50 mM glycine, adjusted to pH 5.0) in a volume ratio 1 : 2 (in further comparative experiments, broader ranges were used).

ECH-Lysine Sepharose 4 Fast-Flow (based on crosslinked 4% agarose, GE Healthcare/Cytiva, USA) was used as stationary phase. This was used on an NGC Chromatography System (BioRad, USA) equipped with 12 mL chromatography column (126 x 11 mm) using a flow-through of 1.2 mL/min (10 min contact time) or a 6 mL chromatography column (63 x 11 mm) using a flow-through of 0.6 mL/min (10 min contact time). The column was equilibrated by a 50 mM sodium acetate buffer of pH 5.0. The solutions containing (Glu-)plasminogen were loaded. The column was washed with a first washing buffer (50 mM sodium acetate, 50 mM glycine, 1 M NaCl, adjusted to pH 5.0). Then, the column was washed with a second washing buffer (10 mM sodium acetate, 50 mM glycine, adjusted to pH 5.0). Finally, the fraction containing Glu-plasminogen was eluted by a citrate-containing elution salt buffer (50 mM citrate, 50 mM glycine, adjusted to pH 3.0).

### g) SDS-PAGE and Western Blot

SDS-PAGE was performed using the Mini-PROTEAN tetra cell system (BioRad, USA) and 10% precast polyacrylamide gels. For a non-reducing SDS-PAGE, the samples were diluted 1 : 4 with Laemmli buffer (non-reducing) and then loaded onto the gel. For a reducing SDS-PAGE, the protein samples were diluted 1 : 4 in Laemmli buffer with 10% dithiothreitol (DTT), and incubated for 5 min at 95 °C. For an SDS-PAGE, the lanes were loaded with 3 µg of protein per well, whereas 1.2 to 2.4 µg of protein per well were used for Western Blots. Each gel was loaded with four standard lanes of Glu-plasminogen, Lys-plasminogen, Lys-plasmin and the Precision Plus Protein All Blue Pre-Stained Protein standard (BioRad, USA). Electrophoresis was carried out at 80 to 200 V with 1x running buffer for ~1 h in a refrigerator at 4 °C. Transfer onto nitrocellulose membranes was performed by wet blotting in 1x Transfer buffer (10x buffer diluted 1:10 with 20% (v/v) methanol) using the Mini Trans-Blot^{®} Electrophoretic Transfer System BioRad, USA) at 350 mA for 1 h. Since the transfer system is set-up outside the refrigerator, a sealed ice unit was added to the buffer tank to cool the system and prevent overheating and temperature fluctuations. Then transfer membranes were blocked in 5% (w/v) dried skimmed milk in TBST buffer for 1 h and incubated overnight with the primary antibody (1 : 700) in 5% (w/v) dried skimmed milk in TBST buffer (tris-buffered saline with Tween-20) at 4 °C. After washing steps using TBST buffer (3 x 5 min), membranes were incubated with a secondary antibody (1 : 2000) conjugated to horseradish peroxidase (in 5% (w/v) dried skimmed milk in TBST buffer) for 1 h at room temperature. Protein bands were detected using SeramunBlau prec^{®} membrane substrate (Seramun Diagnostica GmbH, Germany).

### h) Determining the proteolytic activity

### Total proteolytic activity:

Proteolytic activity (PA) was assessed by monitoring absorption kinetics of samples mixed with the chromogenic substrate S-2288 (100 µL + 100 µL sample) at 450 nm for 1 to 3 min at 37 °C using a spectrophotometer. Samples in the pH range of 7 to 9 were pre-diluted 1 : 4 with PA assay buffer I (0.1 M Tris-HCl 0.106 M NaCl, pH 8.7). For the acidic resuspension samples with a pH below 7, the sample was pre-diluted 1 : 8 with PA assay buffer I to achieve a pH value where the proteases are active again. To meet the linear range of the assay, a dilution ratio of at least 1 : 2 with the PA assay buffer I is used. All buffers used were evaluated regarding the required dilution ratio with PA buffer I to achieve the appropriate target pH of 8.4 for the total proteolytic activity.

### Prevalent proteolytic activity:

To assess the prevalent proteolytic activity of acidic samples in their resuspension environment, an alternative protocol was used. The sample was mixed 1 : 2 with PA assay buffer II (0.025 M Tris-HCl 0.026 M NaCl, pH 8.7). The mixture was then mixed 1 : 2 with the chromogenic substrate S-2288 and the absorption kinetics were measured at 450 nm for 1 to 3 min at 37 °C using a spectrophotometer.

### II) Results

It was surprisingly found that Glu-plasminogen could be obtained at high yields with a very high purity.

In one example, the solubilized protein content (filtrate and wash) after the first dispersion and filtration was 17.6 g having a total proteolytic activity of the first filtrate of 38 IU/g and of 62 IU/g after the first wash.

In this example, the solubilized protein content (filtrate) after the solubilization with acidic buffer was 153 mg. This contained 19.2 mg Glu-plasminogen. Total proteolytic activity in the filtrate was 59 IU/g, prevalent proteolytic activity below detection level. In this context, prevalent proteolytic activity describes the measurement of the converted substrate to measure the currently active but suppressed proteases at the low process pH. For total proteolytic activity, the sample pH was adjusted back to the optimum for substrate conversion and the evaluation of all present proteases that might harm the product.

The recovery rate of cation exchange (CEX) chromatography was 81% (in further comparative experiments, broader ranges were used). The total proteolytic activity dropped from 59 IU/g before CEX to 37 IU/g after CEX. No observable changes in the product composition (Glu/Lys-Plasminogen). For SDS-PAGE/Western Blot (WB) the upper two bands are indicative of Glu-forms (two different glycosylations) and the lower bands are indicative of Lys-forms with their two different glycosylations. Since the lower band of the Glu-forms and the upper band of the Lys-forms overlap, only three bands are visible. In the Western Blot, the commercially available anti-plasminogen antibody "Sheep anti-human Plasminogen (Pg)" ("SAPG-IG") obtained from CoaChrom Diagnostica GmbH (Austria) was fused. An anti-sheep IgG antibody was used as secondary antibody.

**Table 1. Protein contents in different process steps.**

| **Parameter** | **1^{st} OA-PPT resuspension filtrate** | **2^{nd} OA-PPT resuspension filtrate** | **CEX chromatography eluate** | **Affinity chromatography eluate** |
|---|---|---|---|---|
| Total protein conc. (Bradford assay) | 4013 | 381 | 2698 | 462 |
| [µg/mL] | | | | |
| Glu-plasminogen conc. (Glu-plasminogen ELISA*) | Below detection limit | 47 (assumably matrix effect) | 93 | 517 |
| [µg/mL] | | | | |

| | | | | |
|---|---|---|---|---|
| * performed with a kit TECHNOZYM Glu-Plasminogen ELISA Kit (Technoclone GmbH, Austria, #TC12040) | | | | |

In this experiment, at higher protein concentrations, some variation of values was observed. The focus laid on the comparison of samples of different orders of magnitude and within a single experiment, i.e., Bradford assay or ELISA.

The eluate after chromatography with lysine-conjugated Sepharose contained 601 µg/mL of plasminogen of which 587 µg/mL were Glu-plasminogen. This is a purity within the plasminogen content of > 97% (w/w). The chromogenic activity of plasminogen was 164%. The total proteolytic activity was 145 IU/g, the prevalent proteolytic activity below detection level.

In the SDS-PAGE gel (see Figure 6) as well as in the Western Blot (see Figure 7), no plasminogen was detected in the flow-through of the washing buffers before elution (cf. lanes (1) and (2) each).

In the eluate of the affinity chromatography (cf. lanes (13) of Figures 6 and 7), significant amounts of pure (Glu-)plasminogen are obtained. Identity is shown by the Western Blot, purity is shown in the SDS-PAGE gel. All visible impurities were essentially removed. The molecular mass meets the mass of the Glu-plasminogen standard (cf. lanes (5)).

Notably, the flow-through of the affinity chromatography does not show detectible amounts of (Glu-)plasminogen (cf. lanes (14) of Figures 6 and 7). This indicates that the affinity chromatography step does not significantly decrease the obtained yields.

The eluate purity of plasminogen showed a peak of 90.78%. Impurities, which could be detected, are 0.7% (w/w) of albumin, 1.0% (w/w) of immunoglobulin IgG, 0.8% (w/w) of immunoglobulin IgA, and 1.1% (w/w) of immunoglobulin IgM.

**Table 2. Comparison between different sources of the octanoic acid (OA) precipitates obtained from Kistler-Nitschmann precipitate A (PPT-NA) and pastes Kistler-Cohn fractions I+II+III (PPT-KC)**

| Source of the OA-precipitates | CEX chromatography | | Affinity chromatography | Glu-plasminogen product | |
|---|---|---|---|---|---|
| | Step yield (6-AHA spiking) [%] | Protein recovery [%] | Step yield (6-AHA spiking) [%] | Protein recovery [%] | PLG peak area (SEC* analysis) |
| PPT-NA No. 1 | 99 | 81 | 105 | 54 | 90.78 |
| PPT-NA No. 2 | 124 | 75 | 89 | 45 | 89.58 |
| PPT-NA No. 3 | 117 | 77 | 85 | 26 | 90.47 |
| PPT-KC | 84 | 86 | 132 | 67 | 94.55 |

| | | | | | |
|---|---|---|---|---|---|
| * Size Exclusion Chromatography, Enrich SEC 650 10x300 Column 24 mL (BioRad, USA, Art. #780-1650) | | | | | |

The samples were spiked with 6-aminohexanoic acid (6-AHA), i.e., comparable contents of 6-AHA were added in order to improve comparability of the samples with each other.

Accordingly, the method of the present invention is usable with different sources of the octanoic acid (OA) and provides highly reliable high purities of Glu-plasminogen.

## Claims

1. A method for isolating plasminogen from a blood plasma fraction, wherein said method comprises the following steps:
(i) dispersing a precipitate of blood plasma fraction comprising plasminogen in a basic aqueous buffer of pH 7 to 10;
(ii) incubating the dispersion obtained from step (i) to allow the dissolution of at least parts of the proteins and other impurities which are soluble in the basic aqueous buffer;
(iii) separating solid parts and liquid parts of the incubated dispersion of step (ii) from each other;
(iv) mixing the solid parts obtained from step (iii) with an acidic aqueous buffer of pH 2 to 6.6, wherein the acidic aqueous buffer further comprises dissolved lysine and/or at least one other compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, in particular 6-aminohexanoic acid,
and optionally incubating the mixture to allow the plasminogen to dissolve, and optionally removing solid parts; and
(v) obtaining a solution containing isolated plasminogen from step (iv).

2. The method of claim 1, wherein the plasminogen is Glu-plasminogen.

3. The method of any of claims 1 or 2, wherein the blood plasma fraction is selected from the group consisting of:
(a) cryo-poor plasma supernatant or cryo-poor plasma precipitate;
(b) a fraction of any of paste **I,** II or **III** of the Cohn process, or a combination of two or all thereof;
(c) a fraction of any of paste **I,** II or **III** of the Kistler-Nitschmann process, or a combination of two or all thereof; and
(d) a combination of two or all thereof.

4. The method of any of claims 1 to 3, wherein:
the basic aqueous buffer of step (i) is of pH 8.5 to 9.5; and/or
the acidic aqueous buffer in step (iv) is **characterized in that** it is a formic acid, acetate or citrate buffer, **in that** it has pH 4.5 to pH 5.5, or both.

5. The method of any of claims 1 to 4, the step (iii) of separating solid parts and liquid parts from each other is obtained by means selected from the group consisting of filtration, including dead-end filtration, tangential flow filtration, or a combination thereof, dialysis, phase separation, preferably by sedimentation, and/or centrifugation.

6. The method of any of claims 1 to 5, the step (iii) of separating solid parts and liquid parts from each other is obtained by means of filtration, wherein before step (iii), at least one filtration aid is added to the dispersion, preferably wherein the filtration aid is selected from the group consisting polymers, preferably high-molecular weight polyethylene glycol (PEG), cellulose, or cellulose derivatives, diatomaceous earth, perlite, detergents, and combinations of two or more thereof.

7. The method of any of claims 1 to 6, wherein the steps (i) to (iii) are conducted each once, or are repeated twice or more often.

8. The method of any of claims 1 to 7, wherein the dissolved other compound is aminohexanoic acid.

9. The method of any of claims 1 to 8, wherein the solution containing isolated plasminogen obtained in step (v) is further subjected to at least one further step selected from the group consisting of:
contacting the solution with at least one ion exchanger with or without size-excluding properties interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
contacting the solution with at least one size exclusion resin interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
contacting the solution with at least one hydrophobic or mixed-mode interaction chromatography resin interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
subjecting the solution to diafiltration removing the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
subjecting the solution to at least one precipitation-washing cycle;
chromatography based on a stationary phase comprising immobilized lysine and/or at least one immobilized compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine;
affinity chromatography selective for the plasminogen;
molecular size chromatography;
dialysis;
ultrafiltration, including dead-end ultrafiltration, tangential flow ultrafiltration, or a combination thereof; and
a combination of two or more thereof.

10. The method of any of claims 1 to 9, wherein the solution containing isolated plasminogen obtained in step (v) is further subjected to at least the following further steps:
(vi) removing at least parts of the compound comprising one or more amino groups, in particular of the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, from the solution obtained in step (v), preferably by means of contacting the solution with at least one chromatography resin, interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, in particular wherein the chromatography resin is an ion exchanger; and
(vii) increasing the purity and/or the concentration of the plasminogen, preferably by means of performing chromatography based on a stationary phase comprising immobilized lysine and/or at least one immobilized compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, wherein preferably first a buffer allowing the interaction of the plasminogen with the stationary phase is used which is followed by eluting the plasminogen by means of a buffer that decreases the interaction of the plasminogen with the plasminogen; and
(viii) obtaining a solution containing isolated plasminogen from step (vii).

11. The method of any of claims 1 to 10, wherein said method comprises the following steps:
(i) dispersing a precipitate of blood plasma fraction comprising plasminogen in a basic aqueous buffer of pH 7 to 10, preferably pH 8 to 10, more preferably pH 8.5 to 9.5;
(ii) incubating the dispersion obtained from step (i) to allow the dissolution of at least parts of the proteins and other impurities which are soluble in the basic aqueous buffer;
(iii) separating solid parts and liquid parts of the incubated dispersion of step (ii) from each other by means of filtration, including dead-end filtration, tangential flow filtration, or a combination thereof, dialysis, phase separation, preferably by sedimentation, and/or centrifugation;
(iv) mixing the solid parts obtained from step (iii) with an acidic aqueous buffer of pH 2 to 6.6, preferably pH 4.5 to pH 5, wherein the acidic aqueous buffer further comprises dissolved lysine, at least one other compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, or a salt or combination thereof, and optionally incubating the mixture to allow the plasminogen to dissolve, and removing solid parts; and
(v) obtaining a solution containing isolated plasminogen from step (iv);
(vi) removing at least parts of the compound comprising one or more amino groups, in particular of the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, from the solution obtained in step (v), preferably by means of contacting the solution with at least one chromatography resin, interacting with the lysine and/or compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, in particular wherein the chromatography resin is an ion exchanger; and
(vii) increasing the purity and/or the concentration of the plasminogen by means of performing chromatography based on a stationary phase comprising immobilized lysine and/or at least one immobilized compound selected from the group consisting of aminohexanoic acid, aminopentanoic acid, aminoheptanoic acid, aminooctanoic acid, aminononanoic acid, 1,6-diaminohexane, aminodecanoic acid, ornithine, aminomethyl benzoic acid, and oxalysine, wherein first a buffer allowing the interaction of the plasminogen with the stationary phase is used which is followed by eluting the plasminogen by means of a buffer that decreases the interaction of the plasminogen with the plasminogen; and
(viii) obtaining a solution containing isolated plasminogen from step (vii).

12. The method of any of claims 1 to 11, wherein step (iv) and/or step (vi), as far as present, includes contacting the solution with at least one small pore anion exchange resin interacting with at least part of the precipitating agent, in particular octanoic acid.

13. The method of any of claims 1 to 12, wherein:
(a) the proteins which are at least partly dissolved in step (ii) are recovered, preferably wherein said proteins comprise one or more immunoglobulins, in particular one or more fractions of immunoglobulin G;
(b) the method further comprises a virus inactivation step of the fraction of interest;
(c) the pH of the solution containing isolated plasminogen is further adjusted to a desired range, optionally by exchanging the aqueous buffer;
(d) the method further comprises freeze drying or drying of the plasminogen; or
(e) a combination of two or more thereof applies.

14. Use of a basic aqueous buffer of pH 8 to 11 for removing proteins other than plasminogen from a precipitate of a blood plasma fraction comprising plasminogen, wherein the use comprises a method of any of claims 1 to 13.

## Patentansprüche

1. Verfahren zur Isolierung von Plasminogen aus einer Blutplasmafraktion, wobei das Verfahren die folgenden Schritte umfasst:
(i) Dispergieren eines Niederschlags der Blutplasmafraktion, umfassend Plasminogen, in einem basischen wässrigen Puffer mit pH 7 bis 10;
(ii) Inkubieren der aus Schritt (i) erhaltenen Dispersion, um die Auflösung von zumindest Teilen der Proteine und anderer Verunreinigungen, die in dem basischen wässrigen Puffer löslich sind, zu ermöglichen;
(iii) Trennen fester Bestandteile und flüssiger Bestandteile der inkubierten Dispersion aus Schritt (ii) voneinander;
(iv) Mischen der in Schritt (iii) erhaltenen festen Bestandteile mit einem sauren wässrigen Puffer mit pH 2 bis 6,6, wobei der saure wässrige Puffer ferner gelöstes Lysin und/oder mindestens eine weitere Verbindung umfasst, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin, insbesondere 6-Aminohexansäure,
und optional, Inkubieren der Mischung, um dem Plasminogen zu ermöglichen sich zu lösen, und optional, Entfernen fester Bestandteile; und
(v) Erhalten einer Lösung, umfassend isoliertes Plasminogen aus Schritt (iv).

2. Verfahren nach Anspruch 1, wobei das Plasminogen Glu-Plasminogen ist.

3. Verfahren nach einem der Ansprüche 1 oder 2, wobei die Blutplasmafraktion ausgewählt ist aus der Gruppe bestehend aus:
(a) kryo-armem Plasmaüberstand oder kryo-armem Plasmaniederschlag;
(b) einer Fraktion einer der Pasten I, II oder III des Cohn-Prozesses oder einer Kombination aus zwei oder allen davon;
(c) einer Fraktion einer der Pasten I, II oder III des Kistler-Nitschmann-Prozesses oder einer Kombination aus zwei oder allen davon; und
(d) einer Kombination aus zwei oder allen davon.

4. Verfahren nach einem der Ansprüche 1 bis 3, wobei
der basische wässrige Puffer aus Schritt (i) pH 8,5 bis 9,5 aufweist; und/oder der saure wässrige Puffer in Schritt (iv) **dadurch gekennzeichnet ist, dass** er eine Ameisensäure, ein Acetat- oder Citratpuffer ist, dass er pH 4,5 bis 5,5 aufweist, oder beides.

5. Verfahren nach einem der Ansprüche 1 bis 4, wobei der Schritt (iii) des Trennens der festen Bestandteile und der flüssigen Bestandteile voneinander durch Mittel erreicht wird, ausgewählt aus der Gruppe bestehend aus Filtration, einschließlich Dead-End-Filtration, Tangentialflussfiltration oder einer Kombination davon, Dialyse, Phasentrennung, vorzugsweise durch Sedimentation, und/oder Zentrifugation.

6. Verfahren nach einem der Ansprüche 1 bis 5, wobei der Schritt (iii) des Trennens der festen Bestandteile und der flüssigen Bestandteile voneinander mittels Filtration erreicht wird, wobei vor Schritt (iii) mindestens ein Filtrationshilfsmittel zu der Dispersion hinzugefügt wird, vorzugsweise wobei das Filtrationshilfsmittel ausgewählt ist aus der Gruppe bestehend aus Polymeren, vorzugsweise hochmolekularem Polyethylenglykol (PEG), Cellulose oder Cellulosederivaten, Kieselgur, Perlit, Detergentien und Kombinationen von zwei oder mehr davon.

7. Verfahren nach einem der Ansprüche 1 bis 6, wobei die Schritte (i) bis (iii) jeweils einmal durchgeführt oder zweimal oder öfter wiederholt werden.

8. Verfahren nach einem der Ansprüche 1 bis 7, wobei die gelöste andere Verbindung Aminohexansäure ist.

9. Verfahren nach einem der Ansprüche 1 bis 8, wobei die in Schritt (v) erhaltene Lösung, enthaltend isoliertes Plasminogen, ferner mindestens einem weiteren Schritt unterzogen wird, ausgewählt aus der Gruppe bestehend aus:
Inkontaktbringen der Lösung mit mindestens einem Ionenaustauscher mit oder ohne Größenausschluss-Eigenschaften, der interagiert mit dem Lysin und/oder einer Verbindung aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin;
Inkontaktbringen der Lösung mit mindestens einem Größenausschlussharz, das interagiert mit dem Lysin und/oder der Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin;
Inkontaktbringen der Lösung mit mindestens einem hydrophoben oder gemischtmodalen Interaktionschromatographieharz, das interagiert mit dem Lysin und/oder der Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin;
Unterziehen der Lösung einer Diafiltration zum Entfernen des Lysins und/oder der Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin;
Unterziehen der Lösung mindestens einem Ausfällungs-Waschzyklus;
Chromatographie basierend auf einer stationären Phase, umfassend immobilisiertes Lysin und/oder mindestens eine immobilisierte Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin;
Affinitätschromatographie, die für Plasminogen selektiv ist;
Molekülgrößenchromatographie;
Dialyse;
Ultrafiltration, einschließlich Dead-End-Ultrafiltration, Tangentialfluss-Ultrafiltration oder einer Kombination davon; und
einer Kombination aus zwei oder mehr davon.

10. Verfahren nach einem der Ansprüche 1 bis 9, wobei die in Schritt (v) erhaltene Lösung, enthaltend isoliertes Plasminogen, ferner mindestens den folgenden weiteren Schritten unterzogen wird:
(vi) Entfernen von zumindest Teilen der Verbindung, umfassend eine oder mehrere Aminogruppen, insbesondere der Lysin und/oder der Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin, aus der in Schritt (v) enthaltenen Lösung, vorzugsweise mittels Inkontaktbringen der Lösung mit mindestens einem Chromatographieharz, das interagiert mit dem Lysin und/oder der Verbindung, ausgewählt aus der Gruppe bestehend aus Aminohexansäure, Aminopentansäure, Aminooctansäure, Aminononansäure, 1,6-Diaminohexan, Aminodecansäure, , Ornithin, Aminomethylbenzoesäure und Oxalysin, insbesondere wobei das Chromatographieharz ein Ionenaustauscher ist; und
(vii) Erhöhen der Reinheit und/oder der Konzentration des Plasminogens, vorzugsweise mittels Durchführen einer Chromatographie basierend auf einer stationären Phase, umfassend immobilisiertes Lysin und/oder mindestens eine immobilisierte Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin, wobei vorzugsweise zunächst ein Puffer verwendet wird, der die Wechselwirkung des Plasminogens mit der stationären Phase ermöglicht, woraufhin das Plasminogen mittels eines Puffers eluiert wird, der die Wechselwirkung des Plasminogens mit dem Plasminogen verringert; und
(viii) Erhalten einer Lösung, enthaltend isoliertes Plasminogen aus Schritt (vii).

11. Verfahren nach einem der Ansprüche 1 bis 10, wobei das Verfahren die folgenden Schritte umfasst:
(i) Dispergieren eines Niederschlags einer Blutplasmafraktion, umfassend Plasminogen, in einem basischen wässrigen Puffer mit pH 7 bis 10, vorzugsweise pH 8 bis 10, noch bevorzugter pH 8,5 bis 9,5;
(ii) Inkubieren der aus Schritt (i) erhaltenen Dispersion, um die Auflösung von zumindest Teilen der Proteine und anderer Verunreinigungen die in dem basischen wässrigen Puffer löslich sind, zu ermöglichen;
(iii) Trennen fester Bestandteile und flüssiger Bestandteile der inkubierten Dispersion aus Schritt (ii) voneinander mittels Filtration, einschließlich Dead-End-Filtration, Tangentialflussfiltration oder einer Kombination davon, Dialyse, Phasentrennung, vorzugsweise durch Sedimentation, und/oder Zentrifugation;
(iv) Mischen der aus Schritt (iii) erhaltenen festen Bestandteile mit einem sauren wässrigen Puffer mit pH 2 bis 6,6, vorzugsweise pH 4,5 bis 5, wobei der saure wässrige Puffer ferner gelöstes Lysin, mindestens eine weitere Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin, oder ein Salz oder eine Kombination davon umfasst, und, optional, Inkubieren der Mischung, um dem Plasminogen zu ermöglichen sich zu lösen, und Entfernen fester Bestandteile; und
(v) Erhalten einer Lösung, enthaltend isoliertes Plasminogen aus Schritt (iv);
(vi) Entfernen von zumindest Teilen der Verbindung, umfassend eine oder mehrere Aminogruppen, insbesondere des Lysins und/oder der Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin, aus der in Schritt (v) erhaltenen Lösung, vorzugsweise mittels Inkontaktbringen der Lösung mit mindestens einem Chromatographieharz, das interagiert mit dem Lysin und/oder der Verbindung, ausgewählt aus der Gruppe bestehend aus Aminohexansäure, Aminopentansäure, Aminoheptansäure, Aminoctansäure, Aminononansäure, 1,6-Diaminohexan, Aminodecansäure, Ornithin, Aminomethylbenzoesäure und Oxalysin, insbesondere wobei das Chromatographieharz ein Ionenaustauscher ist; und
(vii) Erhöhen der Reinheit und/oder der Konzentration des Plasminogens mittels Durchführen einer Chromatographie basierend auf einer stationären Phase, umfassend immobilisiertes Lysin und/oder mindestens eine immobilisierte Verbindung, ausgewählt aus der Gruppe bestehend aus Aminohexansäure, Aminopentansäure, Aminoheptansäure, Aminooctansäure, Aminononansäure, 1,6-Diaminohexan, Aminodecansäure, Ornithin, Aminomethylbenzoesäure und Oxalysin, wobei zunächst ein Puffer verwendet wird, der die Wechselwirkung des Plasminogens mit der stationären Phase ermöglicht, woraufhin das Plasminogen mittels eines Puffers eluiert wird, der die Wechselwirkung des Plasminogens mit dem Plasminogen verringert; und
(viii) Erhalten einer Lösung, enthaltend isoliertes Plasminogen aus Schritt (vii).

12. Verfahren nach einem der Ansprüche 1 bis 11, wobei Schritt (iv) und/oder Schritt (vi), sofern vorhanden, das Inkontaktbringen der Lösung mit mindestens einem kleinporigen Anionenaustauscherharz einschließt, das interagiert mit zumindest einem Teil des Fällungsmittels, insbesondere Octansäure.

13. Verfahren nach einem der Ansprüche 1 bis 12, wobei:
(a) die Proteine, die in Schritt (ii) zumindest teilweise gelöst werden, zurückgewonnen werden, wobei die Proteine ein oder mehrere Immunglobuline umfassen, insbesondere eine oder mehrere Fraktionen von Immunglobulin G;
(b) das Verfahren ferner einen Schritt zur Inaktivierung von Viren in der Fraktion von Interesse umfasst;
(c) der pH der Lösung, enthaltend isoliertes Plasminogen, ferner auf einen gewünschten Bereich eingestellt wird, optional durch Austausch des wässrigen Puffers;
(d) das Verfahren ferner Gefriertrocknen oder Trocknen des Plasminogens umfasst; oder
(e) eine Kombination von zwei oder mehr davon Anwendung findet.

14. Verwendung eines basischen wässrigen Puffers mit pH 8 bis 11 zum Entfernen von anderen Proteinen als Plasminogen aus einem Niederschlag einer Blutplasmafraktion, umfassend Plasminogen, wobei die Verwendung ein Verfahren nach einem der Ansprüche 1 bis 13 umfasst.

## Revendications

1. Procédé d'isolement de plasminogène à partir d'une fraction de plasma sanguin, dans lequel ledit procédé comprend les étapes suivantes:
(i) la dispersion d'un précipité de fraction de plasma sanguin comprenant du plasminogène dans un tampon aqueux basique de pH 7 à 10;
(ii) l'incubation de la dispersion issue de l'étape (i) pour permettre la dissolution d'au moins des parties des protéines et autres impuretés qui sont solubles dans le tampon aqueux;
(iii) la séparation l'une de l'autre de parties solides et de parties liquides de la dispersion incubée de l'étape (ii);
(iv) le mélange des parties solides obtenues de l'étape (iii) avec un tampon aqueux acide de pH 2 à 6,6, le tampon aqueux acide comprenant en outre de la lysine dissoute et/ou au moins un autre composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine, en particulier l'acide 6-aminohexanoïque,
et éventuellement l'incubation du mélange pour permettre
au plasminogène de se dissoudre, et éventuellement l'élimination de parties solides ; et
(v) l'obtention d'une solution contenant le plasminogène isolé de l'étape (iv).

2. Procédé selon la revendication 1, dans lequel le plasminogène est le Glu-plasminogène

3. Procédé selon l'une quelconque des revendications 1 ou 2, dans lequel la fraction de plasma sanguin est choisie dans le groupe constitué par:
(a) surnageant de plasma cryo-pauvre ou précipité de plasma cryo-pauvre;
(b) une fraction de l'une quelconque parmi la pâte **I,** II ou **III** du procédé Cohn, ou une combinaison correspondante de deux ou de toutes les pâtes;
(c) une fraction d'une pâte quelconque **I,** II ou **III** du procédé Kistler-Nitschmann, ou une combinaison correspondante de deux ou de toutes les pâtes ; et
(d) une combinaison correspondante de deux ou de toutes les pâtes.

4. Procédé selon l'une quelconque des revendications 1 à 3, dans lequel le tampon aqueux basique de l'étape (i) est de pH 8,5 à 9,5; et/ou le tampon aqueux acide de l'étape (iv) est **caractérisé en ce qu'**il s'agit d'un tampon acide formique, acetate ou citrate, **en ce qu'**il présente un pH 4,5 à 5,5, ou les deux.

5. Procédé selon l'une quelconque des revendications 1 à 4, dans lequel l'étape (iii) de séparation de parties solides et de parties liquides les unes des autres est obtenue par un moyen choisi dans le groupe constitué par la filtration, y compris la filtration en bout mort, la filtration à écoulement tangentiel ou une combinaison correspondante, la dialyse, la séparation de phases, de préférence par sédimentation, et/ou la centrifugation.

6. Procédé selon l'une quelconque des revendications 1 à 5, dans lequel l'étape (iii) de séparation de parties solides et de parties liquides les unes des autres est obtenue par filtration, dans lequel, avant l'étape (iii), au moins un adjuvant de filtration est ajouté à la dispersion, de préférence l'adjuvant de filtration étant choisi dans le groupe constitué de polymères, de préférence un polyéthylène glycol (PEG) à haut poids moléculaire, de la cellulose ou des dérivés de cellulose, de la terre de diatomée, de la perlite, des detergents et des combinaisons de deux ou plusieurs correspondantes.

7. Procédé selon l'une quelconque des revendications 1 à 6, dans lequel les étapes (i) à (iii) sont réalisées chacune une fois, ou sont répétées deux fois ou plus souvent.

8. Procédé selon l'une quelconque des revendications 1 à 7, dans lequel l'autre composé dissous est l'acide aminohexanoïque.

9. Procédé selon l'une quelconque des revendications 1 à 8, dans lequel la solution contenant le plasminogène isolé obtenu dans l'étape (v) est en outre soumise à au moins une étape supplémentaire choisie dans le groupe constitué par:
la mise en contact de la solution avec au moins un échangeur d'ions avec ou sans propriétés d'exclusion stérique interagissant avec la lysine et/ou un composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine;
la mise en contact de la solution avec au moins une résine d'exclusion stérique interagissant avec la lysine et/ou un composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine;
la mise en contact de la solution avec au moins une résine de chromatographie d'interaction hydrophobe ou en mode mixte interagissant avec la lysine et/ou un compose choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine;
la soumission de la solution à une diafiltration éliminant la lysine et/ou un composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine ;
la soumission de la solution à au moins un cycle de précipitation-lavage;
la chromatographie basée sur une phase stationnaire comprenant de la lysine immobilisée et/ou au moins un composé immobilisé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine;
la chromatographie d'affinité sélective pour le plasminogène;
la chromatographie par taille moléculaire;
la dialyse;
l'ultrafiltration, y compris l'ultrafiltration en bout mort, l'ultrafiltration à flux tangentiel, ou une combinaison correspondante; et
une combinaison de deux ou plus correspondante.

10. Procédé selon l'une quelconque des revendications 1 à 9, dans lequel la solution contenant le plasminogène isolé obtenue dans l'étape (v) est en outre soumise à au moins les étapes supplémentaires suivantes:
(vi) l'élimination d'au moins des parties du composé comprenant un ou plusieurs groupes amino, notamment de la lysine et/ou du composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine, à partir de la solution obtenue à l'étape (v), de préférence au moyen de la mise en contact de la solution avec au moins une résine de chromatographie, interagissant avec la lysine et/ou un composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine, en particulier dans lequel la résine de chromatographie est un échangeur d'ions; et
(vii) l'augmentation de la pureté et/ou la^concentration du plasminogène, de préférence au moyen de la mise en œuvre d'une chromatographie à base d'une phase stationnaire comprenant de la lysine immobilisée et/ou au moins un composé immobilisé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine, dans lequel, de préférence d'abord un tampon permettant l'interaction du plasminogène avec la phase stationnaire est utilisé qui est suivi d'une élution du plasminogène au moyen d'un tampon qui diminue l'interaction du plasminogène avec le plasminogène; et
(viii) l'obtention d'une solution contenant le plasminogène isolé de l'étape (vii).

11. Procédé selon l'une quelconque des revendications 1 à 10, ledit procédé comprenant les étapes suivantes:
(i) la dispersion d'un précipité de fraction de plasma sanguin comprenant du plasminogène dans un tampon aqueux basique de pH 7 à 10, de préférence de pH 8 à 10, plus préférablement de pH 8,5 à 9,5;
(ii) l'incubation de la dispersion issue de l'étape (i) pour permettre la dissolution d'au moins des parties des protéines et autres impuretés qui sont solubles dans le tampon aqueux basique;
(iii) la séparation de parties solides et de parties liquides de la dispersion incubée de l'étape (ii) l'une de l'autre par filtration, y compris par filtration en bout mort, filtration à flux tangentiel ou une combinaison correspondante, dialyse, séparation de phases, de préférence par sédimentation, et/ou centrifugation;
(iv) le mélange des parties solides obtenues à partir de l'étape (iii) avec un tampon aqueux acide de pH 2 à 6,6, de préférence de pH 4,5 à 5, dans lequel le tampon aqueux acide comprend en outre de la lysine dissoute, au moins un autre composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine, ou un sel ou une combinaison correspondante, et éventuellement l'incubation du mélange pour permettre au plasminogène de se dissoudre, et l'élimination de parties solides ; et
(v) l'obtention d'une solution contenant le plasminogène isolé de l'étape (iv);
(vi) l'élimination d'au moins des parties du composé comprenant un ou plusieurs groupes amino, notamment de la lysine et/ou du composé choisi dans le groupe constitué par l'acide aminohexanoïque, l'acide aminopentanoïque, l'acide aminoheptanoïque, l'acide aminooctanoïque, l'acide aminononanoïque, le 1,6- - 7 - diaminohexane, l'acide aminodécanoïque, l'ornithine, l'acide aminométhylbenzoïque et l'oxalysine, à partir de la solution obtenue à l'étape (v), de préférence au moyen de la mise en contact de la solution avec au moins une résine de chromatographie, interagissant avec la lysine et/ou un composé choisi dans le groupe constitué par l'acide aminohexanoïque, l'acide aminopentanoïque, l'acide aminoheptanoïque, l'acide aminooctanoïque, l'acide aminononanoïque, le 1,6-diaminohexane, l'acide aminodécanoïque, l'ornithine, l'acide aminométhylbenzoïque et l'oxalysine, en particulier dans lequel la résine de chromatographie est un échangeur d'ions; et
(vii) l'augmentation de la pureté et/ou la concentration du plasminogène au moyen de la mise en œuvre d'une chromatographie basée sur une phase stationnaire comprenant de la lysine immobilisée et/ou au moins un composé immobilisé choisi dans le groupe constitué par l'acide aminohexanoïque, l'acide aminopentanoïque, l'acide aminoheptanoïque, l'acide aminooctanoïque, l'acide aminononanoïque, le 1,6- diaminohexane, l'acide aminodécanoïque, l'ornithine, l'acide aminométhylbenzoïque et l'oxalysine, dans lequel d'abord un tampon permettant l'interaction du plasminogène avec la phase stationnaire est utilisé qui est suivi par une élution du plasminogène au moyen d'un tampon qui diminue l'interaction du plasminogène avec le plasminogène; et
(viii) l'obtention d'une solution contenant le plasminogène isolé de l'étape (vii).

12. Procédé selon l'une quelconque des revendications 1 à 11, dans lequel l'étape (iv) et/ou l'étape (vi), dans la mesure où elle est présente, comprend la mise en contact de la solution avec au moins une résine échangeuse d'anions à petits pores interagissant avec au moins une partie de l'agent précipitant, en particulier l'acide octanoïque.

13. Procédé selon l'une quelconque des revendications 1 à 12, dans lequel:
(a) les protéines qui sont au moins partiellement dissoutes dans l'étape (ii) sont récupérées, de préférence dans lequel lesdites protéines comprennent une ou plusieurs immunoglobulines, en particulier une ou plusieurs fractions d'immunoglobuline G;
(b) le procédé comprend en outre une étape d'inactivation virale de la fraction d'intérêt;
(c) le pH de la solution contenant le plasminogène isolé est en outre ajusté jusqu'à une plage souhaitée, éventuellement par échange du tampon aqueux;
(d) le procédé comprend en outre la lyophilisation ou le séchage du plasminogène; ou
(e) une combinaison correspondante de deux ou plusieurs s'applique.

14. Utilisation d'un tampon aqueux basique de pH 8 à 11 pour éliminer des protéines autres que le plasminogène d'un précipité d'une fraction de plasma sanguin comprenant du plasminogène, dans laquelle l'utilisation comprend un procédé selon l'une quelconque des revendications 1 à 13.
